(19) Europäisches Patentamt
European Patent Office
Office européen des brevets

(11) Publication number: **0 066 518**
**B1**

(12) # EUROPEAN PATENT SPECIFICATION

(45) Date of publication of patent specification: **11.06.86**

(21) Application number: **82400990.6**

(22) Date of filing: **27.05.82**

(51) Int. Cl.$^4$: **C 07 C 101/30,**
A 61 K 31/195, C 07 C 99/00,
C 07 C 101/72

(54) **Compounds and methods for treating depression.**

(30) Priority: **01.06.81 US 268553**
**01.06.81 US 268554**

(43) Date of publication of application:
**08.12.82 Bulletin 82/49**

(45) Publication of the grant of the patent:
**11.06.86 Bulletin 86/24**

(84) Designated Contracting States:
**AT BE CH DE FR IT LI LU NL SE**

(56) References cited:
**US-A-4 275 220**

**TETRAHEDRON LETTERS, no. 2, 1979 CHARI et al. "A simple efficient synthesis of beta-methylene phenylalanine. A new approach to the preparation of beta, gamma-unsaturated alpha-amino acid enzyme substrate analogs", pages 111-114**

(73) Proprietor: **MERRELL DOW FRANCE ET CIE**
**16 Rue d'Ankara**
**F-67084 Strasbourg (FR)**

(72) Inventor: **Palfreyman, Michael G.**
**6, Place Forbin**
**F-67640 Fegersheim (FR)**
Inventor: **McDonald, Ian A.**
**13, Rue des Lillas Pfulgriesheim**
**F-67370 Truchtersheim (FR)**

(74) Representative: **Sgarbi, Renato**
**GRUPPO LEPETIT S.p.A.**
**Patent and Trademark Department**
**Via Roberto Lepetit, 8**
**I-20124 Milano (IT)**

**EP 0 066 518 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to pharmacologically-active novel compounds, to methods of preparation of the compounds, and to pharmaceutical compositions containing the compounds.

The class of compounds known as monoamine oxidase inhibitors (MAO inhibitors) has been employed in psychiatry for over 20 years for the treatment of depression [See Goodman and Gilman, *The Pharmacological Basis of Therapeutics*, 6th Ed., McMillan Publishing Co., Inc., N.Y., 1980, pages 427—430]. MAO Inhibitors currently used in the USA for teating depression are tranylcypromine (PARNATE, SKF), phenelzine (NARDIL, Parke-Davis), and isocarboxazid (MARPLAN, Roche). In addition, another MAO inhibitor, pargyline (EUTRON, Abbott), is available for the treatment of hypertension [See *Physicians' Desk Reference,* 34th Ed., Medical Economics Co., Oradell, N.J., 1980, pages 1327—1328 (phenelzine), pages 1466—1468 (isocarboxazid), pages 1628—1630 (tranylcypromine), and pages 521—522 (pargyline)]. MAO Inhibitors can also be employed to treat other psychiatric disorders, such as phobic anxiety states.

It is believed that the MAO inhibitors act to alleviate psychiatric disorders, such as depression, by increasing the concentration of one or more biogenic monoamines in the central nervous system. The monoamine oxidase enzyme (MAO) plays an important role in the metabolic regulation of the monoamines since it catalyzes the biodegradation of the monoamines through oxidative deamination. By inhibiting MAO, the degradation of the monoamines is blocked and the result is an increase in the availability of the monoamines for their physiological functions. Among the physiologically active monoamines which are known substrates for MAO are: (a) so-called "neurotransmitter" monoamines, such as the catecholamines (e.g. dopamine, epinephrine, and norepinephrine) and the indoleamines (e.g. tryptamine and 5-hydroxytryptamine), (b) the so-called "trace" amines (e.g. *o*-tyramine, phenethylamine, *tele-N*-methylhistamine), and (c) tyramine.

The usefulness of the MAO inhibitors in treating depression has been limited because the administration of such agents can potentiate the pharmacological actions of certain food substances or drugs leading to dangerous and sometimes lethal effects. For example, persons receiving a MAO inhibitor must avoid the ingestion of foods which have a high tyramine content (such as cheese) because the MAO inhibitor will block the metabolic degradation of tyramine in the gut and liver resulting in high circulating levels of tyramine, consequent release of catecholamines in the periphery, and finally serious hypertension. The potentiation by a MAO inhibitor of the pressor effect of tyramine arising from the ingestion of cheese, and the hypertensive episode produced thereby, are commonly known as the "cheese reaction" or "cheese effect". Moreover, persons on conventional MAO therapy can not be given directly-acting sympathomimetic drugs (or precursors thereof) which are themselves substrates for MAO (e.g. dopamine, epinephrine, norepinephrine, or L-dopa) and of indirectly-acting sympathomimetic drugs (e.g. amphetamines or over-the-counter cold, hay-fever, or weight control preparations which contain a vasoconstrictor). The potentiation of the pressor effects of indirectly-acting sympathomimetic drugs is especially profound. This is because such drugs act peripherally primarily by releasing catecholamines in nerve endings, and the concentration of the liberated catecholamines will be dangerously elevated if the metabolic degradation of the catecholamines via MAO is blocked. In addition, a particular MAO inhibitor should not be used in combination with another MAO inhibitor or with hypotensive agents, dibenzapine antidepressants, meperidine, CNS depressants, and anticholinergic agents.

Biochemical and pharmacological studies indicate that the MAO enzyme exists in two forms known as "MAO Type A" (MOA—A) and "MAO Type B" (MAO—B). The forms differ in their distribution in body organs, in their substrate specificity, and in their sensitivity to inhibitors. In general, MAO—A selectively oxidizes the so-called "neurotransmitter" monoamines (epinephrine, norepinephrine, and 5-hydroxytryptamine) while MAO—B selectively oxidizes the "trace" monoamines (*o*-tyramine, phenethylamine, and *tele-N*-methylhistamine). Both MAO—A and MAO—B oxidize tyramine, tryptamine, and dopamine. However, in man, dopamine has been shown to be a preferred substrate for MAO—B. The forms also differ in their sensitivity to inhibition, and thus, can be preferentially inhibited depending upon the chemical structure of the inhibitor and/or the relative concentrations of the inhibitor and the enzyme. The MAO inhibitors currently sold in the USA for the therapy of depression (tranylcypromine, phenelzine, and isocarboxazid) are not preferential in their action upon MAO. However, various chemical compounds are known in the art to be preferential inhibitors of MAO, the most important being clorgyline, pargyline, and *L*-deprenyl which are all reported to be clinically effective antidepressant agents. MAO—A is preferentially inhibited by clorgyline, while MAO—B is preferentially inhibited by pargyline and *L*-deprenyl. It should be observed that the "selectivity" of a MAO inhibitor arises because the inhibitor has a greater affinity for one form of the enzyme. Thus, the selectivity of a MAO inhibitor for MAO—A or MAO—B *in vivo* will be dose-dependent, selectivity being lost as the dosage is increased. Clorgyline, pargyline, and *L*-deprenyl are selective inhibitors at lower dosages, but are not selective inhibitors at higher dosages. The literature concerning MAO—A and MAO—B and the selective inhibition thereof is extensive [See, for example, Goodman and Gilman, *ibid,* pages 204—205; Neff *et al, Life Sciences, 14,* 2061 (1974); Murphy, *Biochemical Pharmacology, 27,* 1889 (1978); Knoll, Chapter 10, pages 151—171 and Sandler, Chapter 11, pages 173—181, in *Enzyme Inhibitors as Drugs*, M. Sandler Ed., McMillan Press Ltd., London, 1980; Lipper *et al., Psychopharmacology, 62,* 123 (1979); Mann *et al., Life Sciences, 26,* 877 (1980); and various articles

in *Monoamines Oxidase: Structure, Function, and Altered Functions,* T. Singer *et al.* Ed., Academic Press, N.Y., 1979].

Of the selective inhibitors of MAO, *L*-deprenyl is of interest since the "cheese effect" is not observed at the low dosages where preferential inhibitions of MAO—B occur [See Knoll, *TINS*, pages 111—113, May 1979]. This observation is not unexpected since the intestinal mucosa contains predominantly MAO—A which, because it is not inhibited, permits oxidation and removal of the ingested tyramine. The selectivity of *L*-deprenyl for MAO—B may account for its ability to potentiate L-dopa for the treatment of Parkinson's disease without producing peripheral side effects, such as hypertension due to potentiation of pressor catecholamines [See Lees *et al., Lancet*, pages 791—795, October 15, 1977 and Birkmeyer, *Lancet*, pages 439—443, February 26, 1977].

In a compound aspect, the invention comprehends chemical compounds of the formula:

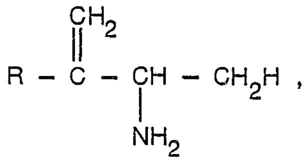 , 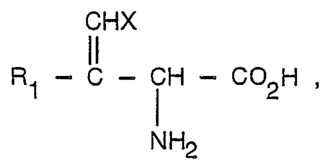 , or 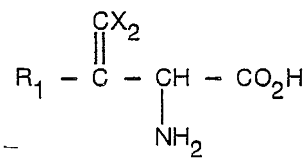

I II III

wherein:

X is fluorine, chlorine, or bromine;

R is the group $R_2$ as defined below;

$R_1$ is a group of the formula:

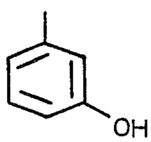 or 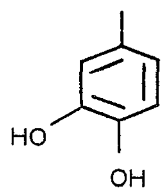

or the group $R_2$ as defined below;
wherein $R_2$ is:

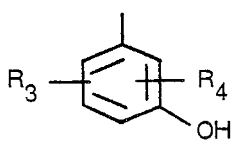 , 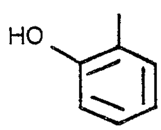 , 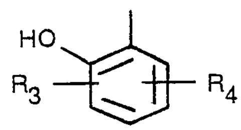 , 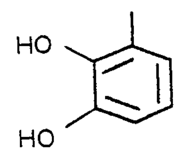 ,

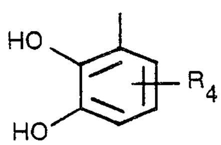 , 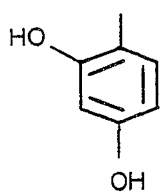 , 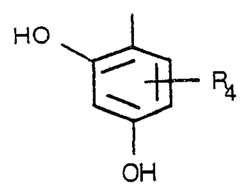 ; 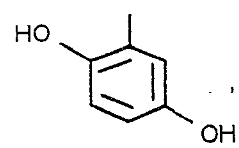 ,

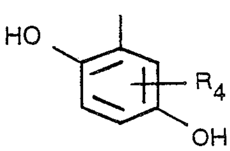 , 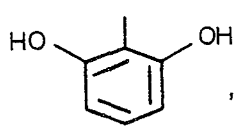 , 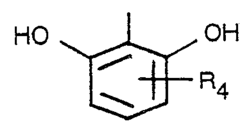 , 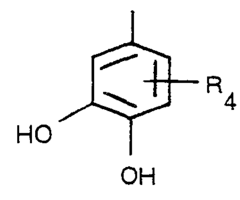 ,

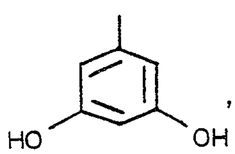 , or 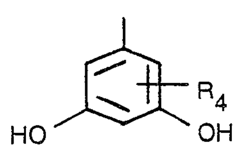 ;

wherein $R_3$ is hydrogen, $(C_1—C_4)$alkyl, or $(C_1—C_4)$alkoxy, and
$R_4$ is $(C_1—C_4)$alkyl or $(C_1—C_4)$alkoxy;
or a non-toxic pharmaceutically acceptable salt thereof.

The term "$(C_1—C_4)$alkyl" contemplates the methyl, ethyl, propyl, *iso*-propyl, *n*-butyl, *tert*-butyl, and *iso*-butyl groups. Methyl is preferred. The term "$(C_1—C_4)$alkoxy" contemplates the methoxy, ethoxy, *n*-propoxy, iso-propoxy, *iso*-butoxy, *tert*-butoxy, and *n*-butoxy groups. Methoxy is preferred. The $(C_1—C_8)$alkyl esters of the compounds of Formula I, II, or III are those wherein the alkyl group forming the ester with the carboxyl group is either straight or branched and contains from 1 to 8 carbons. Examples of esters are methyl, ethyl, propyl, *iso*-propyl, *n*-butyl, *iso*-butyl, *tert*-butyl, *n*-hexyl, *n*-heptyl, or *n*-octyl. The methyl ester is preferred. The ester function must be chosen so that it is capable of being removed to form the free acid *in vivo*.

It will be understood that when $R_2$ is a phenyl group containing one or two —OH groups in combination with other substituents, such as the following phenyl groups:

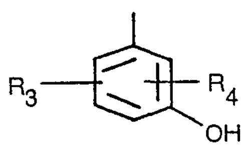 , 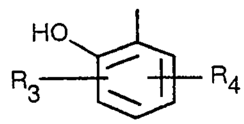 , 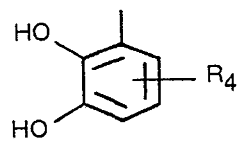 ,

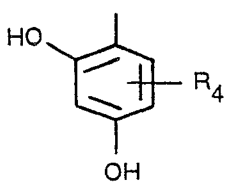 , 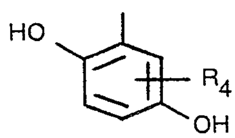 , 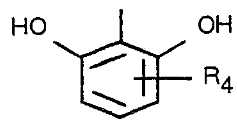 ,

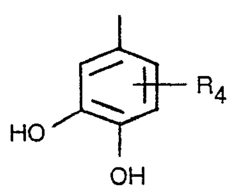 , and 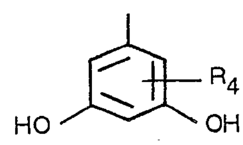 ,

the substituents (represented by $R_3$ or $R_4$) may be located at any of the available positions of the phenyl ring. Illustrative examples of such substituted phenyl groups are:

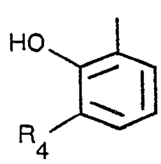 , 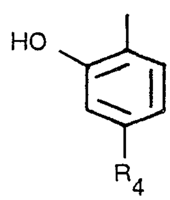 , 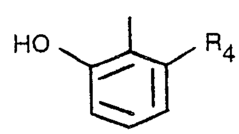 , 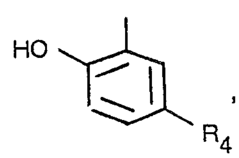 ,

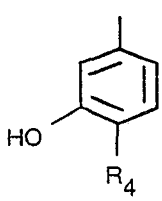 , 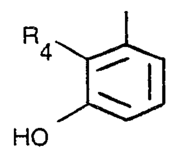 , 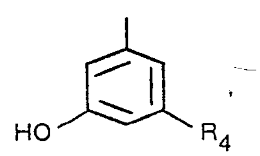 ,

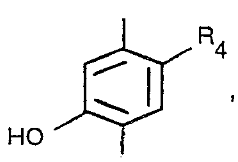 , or 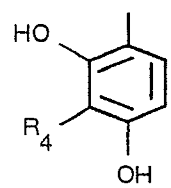 ;

wherein $R_4$ is $(C_1—C_4)$alkyl, $(C_1—C_4)$alkoxy, or fluorine. Preferably R is methyl, ethyl, methoxy, or ethoxy. Methyl is most preferred.

Suitable non-toxic pharmaceutically acceptable salts of the compounds of Formula I, II, or III are known in the art and include acid addition salts formed by protonation of the α-amino group and salts formed by neutralization of the carboxylic acid function. As with any amino acid, the compounds may exist in the form of a zwitterion. Examples of acid addition salts are those formed from the following acids: hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic, and benzenesulfonic. Examples of salts formed by neutralization of the carboxylic acid are metallic salts (e.g. sodium, potassium, lithium, calcium, or magnesium) and ammonium or (substituted) ammonium salts. The potassium and sodium salts are preferred.

Preferred classes of compounds are:

(i) compounds of Formula II or III wherein $R_1$ is 3-hydroxyphenyl.

(ii) compounds of Formula II, or III wherein $R_1$ is

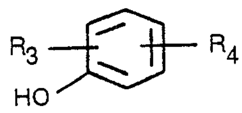 ,

wherein $R_3$ is hydrogen, $(C_1—C_4)$alkyl, or $(C_1—C_4)$alkoxy and $R_4$ is $(C_1—C_4)$alkyl or $(C_1—C_4)$alkoxy.

(iii) compounds as defined in (ii) wherein $R_3$ is hydrogen.

(iv) compounds as defined in Formula II, or III wherein X is fluorine.

(v) compounds of formula II or III as defined in (i), (ii), and (iii) wherein X is fluorine. Preferred embodiments of the compounds of the invention are:

2-amino-3-(3'-hydroxy-4'-methylphenyl)-3-butenoic acid,
2-amino-4-fluoro-3-(2'-hydroxyphenyl)-3-butenoic acid,
2-amino-4-fluoro-3-(3'-hydroxyphenyl)-3-butenoic acid,
2-amino-4-fluoro-3-(2',3'-dihydroxyphenyl)-3-butenoic acid,
2-amino-4-fluoro-3-(2',4'-dihydroxyphenyl)-3-butenoic acid,
2-amino-4-fluoro-3-(2',5'-dihydroxyphenyl)-3-butenoic acid,
2-amino-4-fluoro-3-(2',6'-dihydroxyphenyl)-3-butenoic acid,
2-amino-4-fluoro-3-(3',4'-dihydroxyphenyl)-3-butenoic acid,
2-amino-4-fluoro-3-(3',5'-dihydroxyphenyl)-3-butenoic acid,
2-amino-4-fluoro-3-(3'-hydroxy-4'-methylphenyl)-3-butenoic acid.

The compounds of Formula I, II, or III are *in vivo* precursors (or "prodrugs") of certain substances which are irreversible inhibitors of MAO, and said compounds are useful in psychiatry for the treatment of depression. The compounds of Formula I, II, or III are not irreversible inhibitors of MAO *in vitro*. In order to produce irreversible inhibition of MAO *in vivo* and to exert their antidepressant effect, the compounds of Formula I, II, or III must be transformed into active metabolites which are the 2-phenylallylamine compounds shown below respectively in Formula IV, V, or VI:

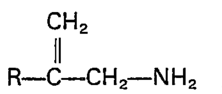 , 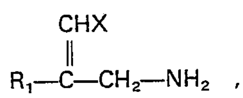 , 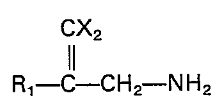

IV V VI

wherein X, R and $R_1$ have the meanings defined *supra*.

The *in vivo* transformation of the compounds of Formula I, II, or III to the active metabolites of Formula IV, V, or VI occurs through a decarboxylation reaction catalyzed by an enzyme known as "aromatic-L-amino acid decarboxylase" (AADC). AADC is known to decarboxylate various biologically important amino acids (such as dopa, tyrosine, phenylalanine, tryptophan, and 5-hydroxytryptophan) to form the corresponding monoamines.

The antidepressant compounds of Formula IV, V, or VI which inhibit MAO *in vitro* and *in vivo* are described and claimed in the pending U.S. application of P. Bey entitled "Allylamine MAO Inhbitors", Serial No. 268,555 filed June 1, 1981.

It is known that AADC is present both in the brain and extracerebral tissue. Thus, the decarboxylation of a compound of formula I, II, or III can take place both in the brain and in extracerebral tissue with consequent inhibition of MAO. By administering a compound of Formula I, II, or III in combination with a compound capable of preferentially blocking extracerebral AADC, the decarboxylation reaction producing the active metabolite will take place primarily in the brain, and hence primarily the brain MAO will be inhibited. The administration of a compound of Formula I, II, or III in combination with a peripheral AADC inhibitor for the treatment of depression, therefore, offers the advantages of substantially avoiding the "cheese effect" and other peripheral complications that are commonly associated with conventional MAO inhibitor therapy. In combination with an extracerebral AADC inhibitor, the compounds of Formula I, II, or

III will provide a "site-directed" inhibition of MAO, the inhibition being confined primarily to the brain which has high AADC activity.

Suitable AADC inhibitors for use in combination with the compounds of Formula I, II, or III will be apparent to those skilled in the art. Both competitive and irreversible inhibitors can be used. At the dosages used, the AADC inhibitor must be capable of inhibiting AADC extracerebrally without substantially inhibiting AADC in the brain. Examples of AADC inhibitors for use in combination with a compound of Formula I, II, or III are carbidopa and benzerazide, compounds which also have been found useful for blocking the peripheral decarboxylation of exogenous L-dopa administered for the treatment of Parkinsonism [See Chapter 21, especially pages 482—483, "*The Pharmacological Basis of Therapeutics*", Goodman and Gilman Ed., McMillan Publishing Co., Inc., N.Y., 6th Ed., 1980]. Other examples of suitable AADC inhibitors are the 2-amino-2-(monofluoromethyl or difluoromethyl)-3-(monohydroxyphenyl or dihydroxyphenyl)propionic acids and like compounds, which are described and claimed in the pending U.S. application of P. Bey and M. Jung entitled "α-Halomethyl Amino Acids", Serial No. 6/210,500 filed November 26, 1980. The aforesaid 2-halomethylated 2-amino-3-(substituted phenyl)propionic acids are also described in Belgian patents Nos. 868,881 and 882,105. Preferred compounds are 2-amino-2-(monofluoromethyl or difluoromethyl)-3-(3′,4′-dihydroxyphenyl)propionic acid, and the 2′,3′- or 2′,5′-dihydroxyphenyl isomers thereof.

The preparation of 2-amino-3-phenyl-3-butenoic acid and 2-amino-3-(3′hydroxyphenyl)-3-butenoic acid from methyl 3-phenyl-2-butenoate and methyl 3-(3′-tetrahydropyranyloxyphenyl)-2-butenoate, respectively, is described by R. Chari in the Doctoral Dissertation entitled "*Synthesis of β, γ- Unsaturated Amino Acids as Potential Irreversible Enzyme Inhibitors*" University of Detroit, 1979 (available in print from University Microfilm International, Ann Arbor, Mich.). The compounds of Formula I in general can be prepared according to the method Chari, or a modification thereof, depending upon the type of substitution in the phenyl ring and the consequent sensitivity of the double bond to acid. The starting materials for the preparation of the compounds of Formula I are the alkyl 2-butenoate compounds of Formula VII:

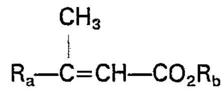

VII

wherein:

$R_b$ is $(C_1—C_4)$alkyl, and

$R_a$ is a group of the formula:

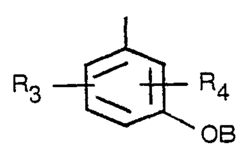 , 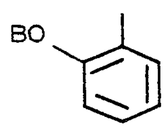 , 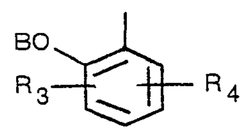 ,

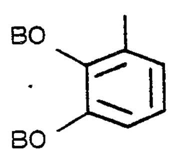 , 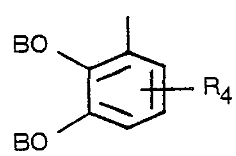 , 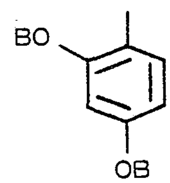 ,

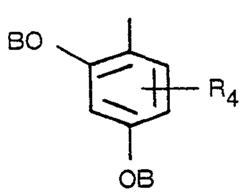 , 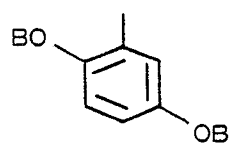 , 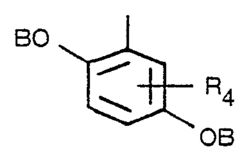 ,

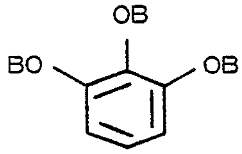 , 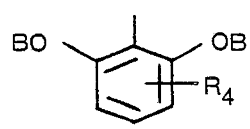 , 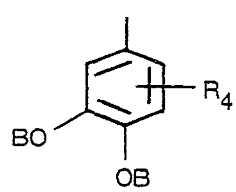 ,

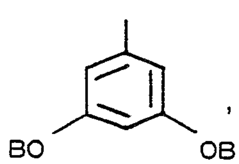 , or 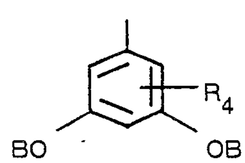 ;

wherein:

$R_3$ and $R_4$ have the meanings defined *supra* and

B is a protecting group for an aromatic —OH group.

The preferred starting materials of Formula VII are those wherein $R_b$ is methyl, ethyl, or *tert*-butyl. The purpose of the group defined by $R_b$ is to protect the carboxy group from unwanted side reactions during subsequent process steps. The group represented by B in the definition of $R_a$ can be any group known to be useful in the art of chemistry for protecting an aromatic —OH group, is readily removable under conditions which will not cause side reactions (such as polymerization) involving the double bond. Suitable protecting groups are: tetrahydropyranyl, methoxymethyl, methoxyethoxy-methyl, *tert*-butyl, benzyl, or triphenylmethyl. Preferred protecting groups are those that can be removed under very mild conditions. The selection and utilization of a particular aromatic —OH protecting group are known *per se* in the art.

In the method of Chari, a compound of Formula VII is brominated in carbon tetrachloride at −10°C to form the corresponding alkyl dibromobutyrate compound of formula VIII:

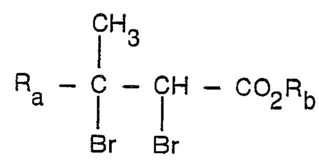

VIII

which upon treatment with ammonia in dimethyl sulfoxide (DMSO) affords an alkyl 2-amino-3-butenoate compound of Formula IX:

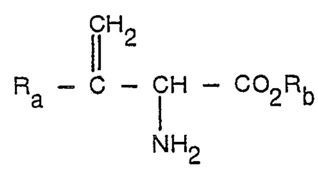

IX

When the intermediate of Formula IX does not have a protected —OH group or an alkoxy group in the 2- and/or 4-position of the phenyl ring, the intermediate can be converted to a compound of Formula I in two stages: (a) treatment with an acid (preferably dilute hydrochloric acid or saturated ethereal hydrogen chloride) under mild conditions (a temperature of 0 to 25°C) for up to 16 days under which conditions the aromatic —OH protecting group (B) is removed, and (b) treatment with a strong acid (6N hydrochloric acid) at reflux temperature under which conditions the ester group is hydrolyzed.

When the intermediate of Formula IX has a protected aromatic —OH group or an alkoxy group in the 2- and/or 4-position of the phenyl ring, the intermediate can be converted to a compound of Formula I by a three-stage process which comprises:

(a) alkaline hydrolysis (preferably with lithium hydroxide in dioxane/water) at ambient temperature to remove the ester alkyl group ($R_b$),

(b) neutralization of the salt thus formed (to pH *ca* 4.0, preferably with dilute hydrochloric acid) to afford the corresponding free acid, and

(c) treatment with an acid (preferably dilute hydrochloric acid or ethereal hydrogen chloride) under mild conditions (a temperature of 0 to 25°C) for up to 16 hours to remove the aromatic —OH protecting group (B).

When $R_b$ in formula IX is *tert*-butyl, steps (a) and (b) can be omitted. With acid sensitive intermediates, the three step process is preferred because the use of basic conditions and/or mild acid conditions to hydrolyze the ester function and to remove the protecting groups will minimize the occurrence of side reactions involving the double bond (e.g. polymerization) which reactions are promoted under vigorous acidic conditions.

A modification of the above-described method involves the intermediate preparation and isolation of an N-protected 2-amino-3-butenoate compound of Formula X:

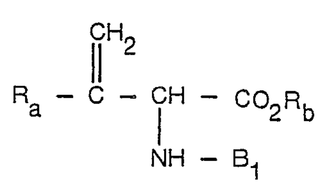

X

wherein:

$R_a$ and $R_b$ have the meanings defined *supra*, and

$B_1$ is a protecting group for a primary α-amino group.

The group represented by $B_1$ can be any group known in the art to be useful for protecting a primary α-amino group, which group is readily removable under acidic conditions which will not cause side reactions (such as polymerization) involving the double bond. The selection of a particular protecting group and its method of utilization are known in the art. Examples of suitable protecting groups for the α-amino group are: formyl, acetyl, trifluoroacetyl, phthalyl, tosyl, benzenesulfonyl, benzyloxycarbonyl, (substituted)-benzyloxycarbonyl, (e.g. the *p*-chloro, *p*-bromo, *p*-nitro, *p*-methoxy, *o*-chloro, 2,4-dichloro, and 2,6-dichloro derivatives), t-butyloxycarbonyl (Boc), t-amyloxycarbonyl, isopropyloxycarbonyl, 2-(p-biphenyl)-isopropyloxycarbonyl, allyloxycarbonyl, cyclopentyloxycarbonyl, cyclohexyloxycarbonyl, adamentyloxy-carbonyl, phenylthiocarbonyl, or triphenylmethyl. The preferred α-amino protecting group is *t*-butyloxycarbonyl (Boc) which can be introduced by reaction with di-*tert*-butyl dicarbonate.

The intermediate for Formula X can be prepared by treating a compound of Formula IX, as prepared according to the above-described method of Chari, with di-*tert*-butyl dicarbonate in tetrahydrofuran at 60°C for about 2—4 hours. The intermediate of Formula X, regardless of the type of substitution in the phenyl ring can be converted to a compound of Formula I by a three-stage procedure which comprises:

(a) alkaline hydrolysis (preferably with lithium hydroxide in dioxane/water) at ambient temperature to remove the ester alkyl group ($R_b$),

(b) neutralization of the salt thus formed (to pH *ca* 4.0, preferably with dilute hydrochloric acid) to afford the corresponding free acid, and

(c) treatment with an acid (preferably dilute hydrochloric acid or ethereal hydrogen chloride) under mild conditions (a temperature of 0 to 25°C) for up to 16 hours to remove the aromatic —OH protecting group (B) and the α-amino group ($B_1$). When $R_b$ in Formula X is *tert*-butyl, steps (a) and (b) can be omitted.

The modified method, as described above, is especially preferred for making the compounds of Formula I which have the —OH group or an alkoxy group at the 2-position or 4-position of the phenyl ring. Use of the N-protected derivative of Formula X in the modified procedure rather than the free amine intermediate of Formula IX, avoids decomposition of the amino acid during the subsequent base hydrolysis step and aids in purification of the hydrolysis product.

The compounds of Formula II can be prepared in manner known *per se* by the method depicted below:

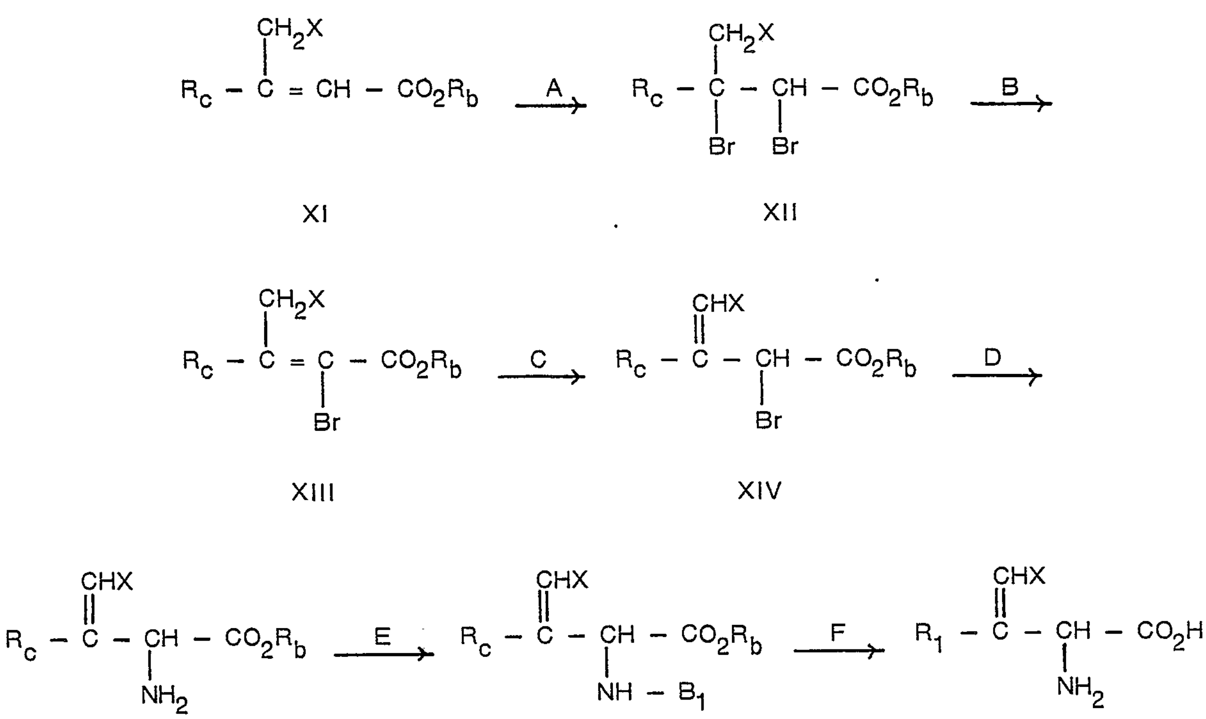


XI XII XIII XIV XV XVI II

in the above reaction scheme, $R_c$ is a group of the formula:

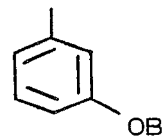

or

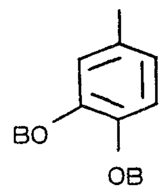

,

or a group as defined by $R_a$ in Formula VII; $R_b$ is $(C_1—C_4)$alkyl; $B_1$ is a protecting group for a primary α-amino group; and X is fluorine, chlorine, or bromine except that the protecting group (B), as defined by $R_c$ or $R_a$, cannot be tetrahydropyranyl. In *Step A*, an alkyl 4-halo-2-butenoate compound of Formula XI is brominated in manner known *per se*, preferably in carbon tetrachloride at 0°C, to afford an alkyl 2,3-dibromo-4-halobutenoate compound of Formula XII. In *Step B*, the compound of Formula XII is dehydrohalogenated in manner known *per se*, preferably by treatment with sodium hydride in tetrahydrofuran (THF) at reflux temperature, to give the alkyl 4-halo-2-bromo-2-butenoate compound of Formula XIII, which is then isomerized (step C) in manner known *per se* to the corresponding 3-butenoate compound of Formula XIV, preferably by treatment with lithium diisopropylamine in THF at −78°C. In *Step D*, the 3-butenoate compound of Formula XIV is treated with ammonia, preferably in dimethylsulfoxide (DMSO), at ambient temperature to afford the alkyl 4-halo-2-amino-3-butenoate compound of Formula XV, which in *Step E* is treated in manner known *per se* to form the N-protected derivative of Formula XVI. The conversion (*Step F* of the N-protected derivative of Formula XVI to a final product of Formula II can be accomplished by a three-stage procedure which comprises:

(a) alkaline hydrolysis (preferably with lithium hydroxide in dioxane/water) at ambient temperature to remove the ester alkyl group ($R_b$),

(b) neutralization of the salt thus formed (to pH *ca* 4.0, preferably with dilute hydrochloric acid) to afford the corresponding free acid, and

(c) treatment with an acid (preferably dilute hydrochloric acid or ethereal hydrogen chloride) under mild conditions (a temperature of 0 to 25°C) for up to 16 hours to remove the aromatic —OH protecting group (B) and the α-amino group ($B_1$). When $R_b$ in Formula X is *tert*-butyl, steps (a) and (b) can be omitted.

When it is desired to prepare a compound of Formula II in which the —OH group is present at the 3-position of the phenyl ring, and a —OH or alkoxy is *not* present in the 2- or 4-position of the phenyl ring, a further modified procedure can be used. This procedure is similar to that described above for preparing the compounds of Formula II, except that the starting material is a compound of Formula XI in which the aromatic —OH protecting group (B), as defined by $R_a$ can also be a $(C_1—C_4)$straight chain alkyl group. The preparation is carried out in a manner similar to that described above for Steps A, B, C, D, and E. However, the N-protected derivative formed in *Step E* can be converted to a compound of Formula I in one step by treatment with 47% hydrobromic acid at reflux temperature. Such treatment will remove the aromatic —OH protecting group (B), remove the ester alkyl function ($R_b$), and remove the α-amino protecting group ($B_1$). Alternatively the N-protected derivative can be treated with dilute hydrochloric acid or saturated ethereal hydrogen chloride to remove the α-amino protecting group before treatment with 47% hydrogen bromide.

In *Step E*, the preferred N-protecting group is *tert*-butoxycarbonyl (Boc) which can be introduced into a compound of Formula XV in manner known *per se*, such as by reaction with di-*tert*-butyl dicarbonate.

The compounds of Formula III can be prepared in a manner similar to that described for preparing the compounds of Formula II using as the starting material a compound of Formula XVII:

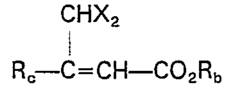

XVII

wherein $R_b$, $R_c$, and X have the meanings defined *supra* with respect to Formula XI.

The starting material of Formula VII, XI, or XVII can be prepared in known manner by the Wittig reaction by treating a ketone of the Formula:

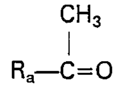 , 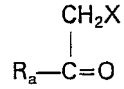 , or 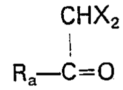 ,

XVIII XIX XX

with a suitable trialkylphosphonoacetate in dimethoxyethane (DME) at 0°C in the presence of sodium hydride. The ketones of Formula XVIII, XIX, or XX are either known compounds or they can be prepared from known compounds by methods known in the art or obvious modifications thereof. For example, the

compounds of Formula XVIII can be halogenated in known manner to prepare the compounds of Formula XIX or XX or an appropriately substituted benzene compound can be acylated using a Friedel-Craft reaction.

As will be appreciated by those skilled in the art, the compounds of Formula I, II, or III have an aromatic —OH group and an *alpha*-$NH_2$ group, one or both of which may be acylated in manner known *per se*. It is known in the art that the N-acyl or O-acyl groups derived from an alkanoic acid or a naturally occurring amino acid can be removed to generate the free —$NH_2$ group or —OH group *in vivo*. Thus, the acyl derivatives can also be employed for the purposes of this invention, provided that the acyl group can be removed *in vivo* to give the desired amino acid. It will also be recognized that certain other derivatives can be converted *in vivo* to generate a free aromatic hydroxyor α-amino group.An example of such a derivative is 2-amino-4-fluoro-3-(3',4'-methylenedioxyphenyl)-3-butenoic acid. It will also be apparent that certain derivatives of the carboxylic acid function, other than esters and salts, can be employed for the purposes of this invention. Examples are primary amides, secondary or tertiary alkyl amides, and amides formed from the *alpha*- or terminal $NH_2$— group of natural amino acids, since it is known in the art that the amide bond can be cleaved *in vivo*.

Since the compounds of Formula I, II, or III possess an asymetric carbon atom enantiomers are possible, and the compounds of the invention may be in the form of the biologically active enantiomer or the recemate.

The compounds of Formula I, II, or III may be obtained in the form of a pure enantiomer either by resolving a desired racemic product or by resolving a racemic starting material or intermediate at any convenient stage of the synthesis. Methods of carrying out the resolution are well known in the art of chemistry. When dosage ranges are given herein, they are applicable to the racemate.

In addition, the compounds of Formula II can exist in forms wherein the substituents represented by X can be either *cis* or *trans* to the group represented by $R_1$. It is understood that the compounds of the invention may exist as the pure *cis* or *trans* form, or as mixtures thereof.

The structural formula of 2-amino-3-(3'-hydroxyphenyl)-3-butenoic acid and 2-amino-3-(3',4'-dihydroxyphenyl)-3-butenoic acid are shown below in Formula XXI and XXII, respectively:

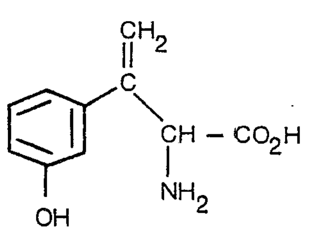

XXI

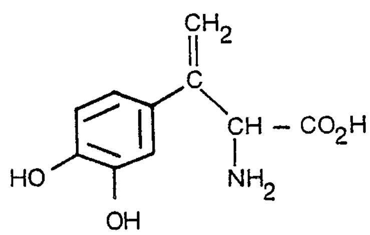

XXII

The ($C_1$—$C_8$)alkyl esters and the pharmaceutically acceptable salts of compounds XXI and XXII can be made by conventional methods.

Suitable non-toxic, pharmaceutically acceptable salts are known in the art and include acid addition salts formed by protonation of the α-amino group and salts formed by neutralization of the carboxylic acid function. As with any amino acid, the compounds may exist in the form of a zwitterion. Examples of acid addition salts are those formed from the following acids: hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic, and benzenesulfonic. Examples of salts formed by neutralization of the carboxylic acid are metallic salts (e.g. sodium, potassium, lithium, calcium, or magnesium) and ammonium or (substituted) ammonium salts. The potassium and sodium salts are preferred.

Compounds XXI and XXII are metabolic precursors (or "prodrugs") of substances which are irreversible inhibitors of MAO, and said compounds are useful in psychiatry for the treatment of depression. Compounds XXI and XXII are not irreversible inhibitors of MAO *in vitro*. In order to produce irreversible inhibition of MAO *in vivo*, and to exert their antidepressant effect, compounds XXI and XXII must be transformed into active metabolites which are the 2-phenylallylamine compounds shown below respectively in Formula XXIII and XXIV:

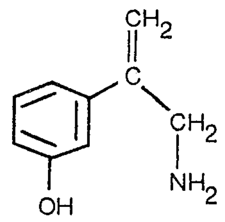

XXIII

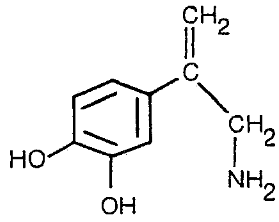

XXIV

The *in vivo* transformation of compounds XXI or XXII to the active metabolites of Formula XXIII or XXIV occurs through a decarboxylation reaction catalyzed by an enzyme known as "aromatic-L-amino acid decarboxylase" (AADC). AADC is known to decarboxylate various biologically important amino acids (such as dopa, tyrosine, phenylalanine, tryptophan, and 5-hydroxytryptophan) to form the corresponding monoamines.

The antidepressant compounds of Formula XXIII or XXIV, which inhibit MAO *in vitro* and *in vivo*, are described and claimed in the pending U.S. application of I. McDonald and M. Palfreyman entitled "Method for Treating Depression", Serial No. 268,554 filed June 1, 1981.

It is known that AADC is present both in the brain and extracerebral tissue. Thus, the decarboxylation of compounds XXI or XXII can take place both in the brain and in extracerebral tissue with consequent inhibition of MAO. By administering compounds XXI or XXII in combination with a compound capable of preferentially blocking extracerebral AADC, the decarboxylation reaction producing the active metabolite will take place primarily in the brain, and hence primarily the brain MAO will be inhibited. The administration of compound XXI or XXII in combination with a peripheral AADC inhibitor for the treatment of depression, therefore, offers the advantages of substantially avoiding the "cheese effect" and other peripheral complications that are commonly associated with conventional MAO inhibitor therapy. In combination with an extracerebral AADC inhibitor, compound XXI or XXII will provide a "site-directed" inhibition of MAO, the inhibition being confined primarily to the brain which has high AADC activity.

Suitable AADC inhibitors for use in combination with the compounds of Formula XXI and XXII will be apparent to those skilled in the art. Both competitive and irreversible inhibitors can be used. At the dosages used, the AADC inhibitor must be capable of inhibiting AADC extracerebrally without substantially inhibiting AADC in the brain. Examples of AADC inhibitors for use in combination with a compound of Formula XXI and XXII are carbidopa and benzerazide, compounds which also have been found useful for blocking the peripheral decarboxylation of exogenous L-dopa administered for the treatment of Parkinsonism [See Chapter 21, especially pages 482—483, *"The Pharmacological Basis of Therapeutics"*, Goodman and Gilman Ed., McMillan Publishing Co., Inc., N.Y., 6th Ed., 1980]. Other examples of suitable AADC inhibitors are the 2-amino-2-(monofluoromethyl or difluoromethyl)-3-(monohydroxyphenyl or dihydroxyphenyl)propionic acids and like compounds, which are described and claimed in the pending U.S. application of P. Bey and M. Jung entitled "α-Halomethyl Amino Acids", Serial No. 6/210,500 filed November 26, 1980. The aforesaid 2-halomethylated 2-amino-3-(substituted phenyl)-propionic acids are also described in Belgian patents Nos. 868,881 and 882,105. Preferred compounds are 2-amino-2-(monofluoromethyl or difluoromethyl)-3-(3′,4′-dihydroxyphenyl)propionic acid, and the 2′,3′- or 2′,5′-dihydroxyphenyl isomers thereof.

The preparations of 2-amino-3-(3′-hydroxyphenyl)-3-butenoic acid (XXI) from methyl 3-(3′-hydropyranyloxyphenyl)-2-butenoate is described by R. Chari in the Doctoral Dissertation entitled *"Synthesis of β,γ-Unsaturated Amino Acids as Potential Irreversible Enzyme Inhibitors"*, University of Detroit, 1979 (available in print from University Microfilm International, Ann Arbor, Mich.).

In the method of Chari, methyl 3-(3′-tetrahydropyranyloxyphenyl)-2-butenoate is brominated in carbon tetrachloride at −10°C to form the corresponding dibromo compound which upon treatment with ammonia in dimethyl sulfoxide (DMSO) affords methyl 2-amino-3-(3′-tetrahydropyranyloxyphenyl)-3-butenoate. This intermediate can be converted to compound XXI in two stages: (a) treatment with saturated ethereal hydrogen chloride at ambient temperature, under which conditions the aromatic —OH protecting group is removed, and (b) treatment with 6N hydrochloric acid at reflux temperature under which conditions the ester group is hydrolyzed.

A modification of the above-described method involves the preparation and isolation of ethyl 2-(*tert*-butoxycarbonylamino)-3-(3′-tetrahydropyranyloxyphenyl)-3-butenoate. This intermediate is prepared by treating methyl 2-amino-3-(3′-tetrahydropyranyloxyphenyl)-3-butenoate, as prepared according to the above-described method of Chari, with di-*tert*-butyl dicarbonate in tetrahydrofuran at 60°C for about 2—4 hours. The intermediate can be converted to compound I by a three-stage procedure which comprises:

(a) treating the *N-tert*-Boc derivative with lithium hydroxide in dimethoxyethane/water at ambient temperature to hydrolyze the ester function,

(b) neutralizing the lithium salt then formed with dilute hydrochloric acid (to pH *ca* 4.0) to form the corresponding free acid, and

(c) treating the free acid with saturated ethereal hydrogen chloride at 0 to 25°C for about 16 hours to remove the *tert*-Boc group.

The modified procedure for preparing 2-amino-3-(3′-hydroxyphenyl)-3-butenoic acid is described in Example 13 herein. 2-Amino-3-(3′,4′-dihydroxyphenyl)-3-butenoic acid (XXII) can be prepared from a suitably protected ethyl 3-(3′,4′-dihydroxyphenyl)-2-butenoate by following the general procedure of Example 13. However, the aromatic hydroxy protecting groups must be removed under very mild conditions to avoid the occurrence of side reactions involving the double bond. The starting materials for the bromination reaction can be prepared in known manner by the Wittig reaction by treating the appropriate —OH protected ketone with a suitable trialkylphosphonoacetate in DME at 0°C in the presence of sodium hydride. The ketones are known compounds. Example 12 herein illustrates the preparation of ethyl 3-(3′-tetrahydropyranyloxy-phenyl)-2-butenoate from 3-tetrahydropyranyloxy-acetophenone.

As will be appreciated by those skilled in the art, compounts XXI and XXII have an aromatic —OH group and an *alpha*-$NH_2$ group, one or both of which may be acylated in manner known *per se*. It is known in the art that the N-acyl or O-acyl groups derived from an alkanoic acid or a naturally occurring amino acid can be metabolically removed to generate the free —$NH_2$ group or —OH group *in vivo*. Thus, the acyl derivatives can also be employed for the purposes of this invention, provided that the acyl group can be removed *in vivo* to give the desired amino acid. It will also be apparent that certain derivatives of the carboxylic acid function, other than esters and salts, can be employed for the purposes of this invention. Examples are primary amides, secondary and tertiary alkyl amides, and amides formed from the *alpha*- or terminal $NH_2$— group of natural amino acids, since it is known in the art that the amide bond can be cleaved metabolically.

Since compounds XXI and XXII possess an asymetric carbon atom, enantiomers are possible, and the compounds may be in the form of the biologically active enantiomer or the racemate. The compounds may be obtained in the form of a pure enantiomer either by resolving a desired racemic product or by resolving a racemic starting material or intermediate at any convenient stage of the synthesis. Methods of carrying out the resolution are well known in the art of chemistry. When dosage ranges are given herein, they are applicable to the racemate.

In a process aspect, the invention provides the following methods of preparation:

(1) A method for preparing a compound of Formula I, II, or III which comprises hydrolyzing a compound of the formula:

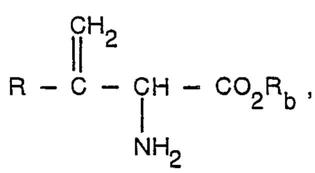, 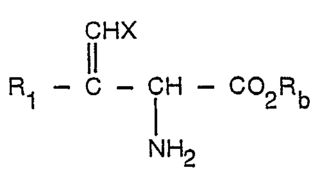, or 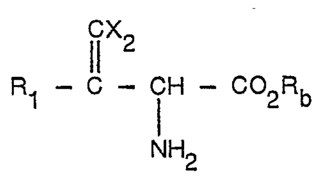

XXIII XXIV XXV

wherein:

X is fluorine, chlorine, or bromine,

$R_b$ is $(C_1—C_4)$alkyl,

R is the group $R_2$, as defined below,

$R_1$ is the group $R_2$, as defined below, or a group of the formula:

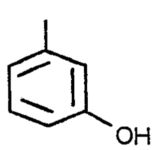 or 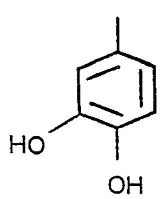

and $R_2$, as defined by R or $R_1$, above, is

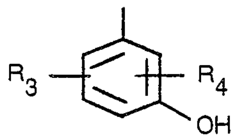 , 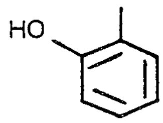 , 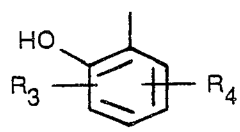 ,

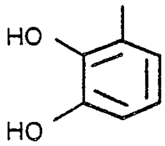 , 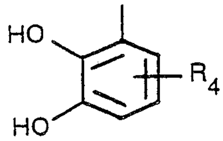 , 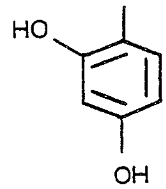 ,

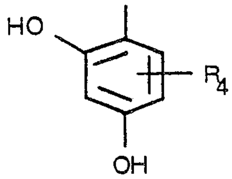 , 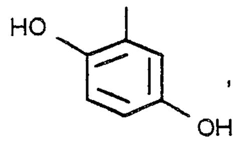 , 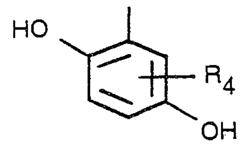 ,

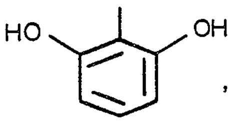 , 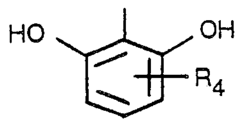 , 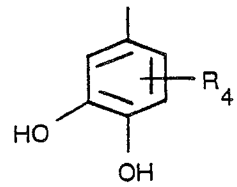 ,

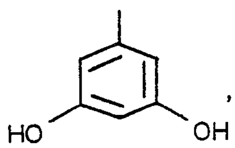 , or 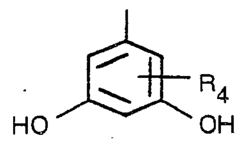 ;

wherein

$R_3$ is hydrogen, $(C_1—C_4)$alkyl, or $(C_1—C_4)$alkoxy, and

$R_4$ is $(C_1—C_4)$alkyl or $(C_1—C_4)$alkoxy;

or an aromatic hydroxy-protected and/or amino-protected derivative thereof; and, if necessary, removing in manner known *per se* the protecting group(s) either simultaneously with or subsequent to the hydrolysis reaction.

(2) A method as defined in (1) above wherein said compound treated is an aromatic hydroxy-protected derivative.

(3) A method as defined in (1) above wherein said compound treated is an amino-protected derivative.

(4) A method as defined in (1) above wherein said compound treated is an aromatic hydroxy-protected and amino-protected derivative.

(5) A method as defined in (1) above wherein said compound treated is a compound of the formula:

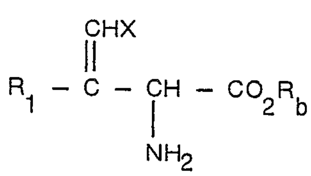 or 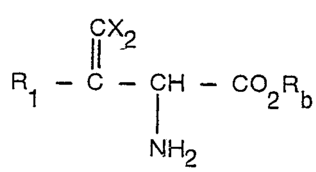

XXIV XXV

wherein:

$R_b$ is $(C_1—C_4)$alkyl,

X is fluorine, chlorine, or bromine, and

$R_1$ is

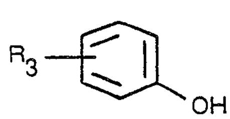

wherein:

$R_3$ is hydrogen or $(C_1—C_4)$alkyl; or an aromatic hydroxy-protected and/or amino-protected derivative thereof.

(6) A method as defined in (5) wherein X is fluorine and $R_3$ is 3-hydroxyphenyl.

(7) A method as defined in (6) wherein the compound treated is a $(C_1—C_4)$alkyl ester of 2-amino-4-fluoro-3-(3'-hydroxyphenyl)-3-butenoic acid, or an aromatic hydroxy-protected and/or amino acid protected derivative thereof.

(8) A method as defined in (5), (6), or (7) wherein the hydrolysis reaction is carried out using an aqueous strong acid at reflux temperature, under which conditions an aromatic hydroxy-protecting group or amino-protecting group is removed.

(9) A method as defined in (8) wherein the hydrolysis is carried out using 47% aqueous hydrogen bromide at reflux temperature.

When employed to treat depression, the effective dosage of the compounds of Formula I, II, III, XXI, or XXII will vary according to the particular compound being employed, the severity and nature of the depression, and the particular subject being treated. In general, with the compounds of Formula I, XXI, or XXII, effective results can be achieved by the oral or parenteral route at a dosage level of from about 20 to about 200 mg per day, while with the compounds of Formula II and III, effective results can be achieved by the oral or parenteral route at a dosage level of from about 0.5 to about 50 mg per day.

Therapy should be initiated at lower dosages, the dosage thereafter being increased until the desired effect is achieved.

When an AADC inhibitor is co-administered with a compound of Formula I, II, III, XXI, or XXII, for the treatment of depression, the effective dosage of the AADC inhibitor must be capable of substantially blocking the AADC catalyzed decarboxylation of said compound extracerebrally without substantially blocking the AADC catalyzed decarboxylation in the brain. The effective dose will vary, however, according to the particular compound being employed and the dose of the antidepressant "prodrug" administered. In general, with carbidopa and benzerazide effective results can be achieved by the oral or parenteral route at a dosage level of about 50 to 500 mg per day, preferably about 50 to 250 mg. With the 2-halomethylated 2-amino-3-(substituted phenyl)propionic acids described *supra*, effective results can be achieved by the oral or parenteral route at a dosage level of about 0.1 mg to 1000 mg per day. For example, with 2-amino-2-difluoromethyl-3-(3',4'-dihydroxyphenyl)propionic acid, and like compounds, the effective dose is about 10 to 1000 mg per day, preferably about 100 to 500 mg. With 2-amino-2-fluoromethyl-3-(3',4'-dihydroxyphenyl)propionic acid, and like compounds, such as the 2,3-dihydroxyphenyl isomer thereof, the effective dose is about 0.1 to 50 mg per day, preferably about 0.5 to 10 mg.

It will be understood that the AADC inhibitor can be co-administered either substantially at the same time as or prior to the administration of a compound of Formula I, II, III, XXI, or XXII. When administered prior, the AADC inhibitor can be given up to 4 hours prior, depending upon the route of administration and severity of the conditions being treated.

When used in combination with an AADC inhibitor, a compound of Formula I, II, III, XXI, or XXII and the AADC inhibitor can be administered separately, each being contained in a formulation in which the compound or the AADC inhibitor is the sole active agent or they can be administered together in a formulation containing both the compound and the AADC inhibitor as active agents. When both agents are contained in a single formulation, the relative amounts of each agent can vary depending upon the particular compounds employed.

The compounds of this invention can be administered in various manners to achieve the desired effect. The compounds can be administered alone or in combination with pharmaceutically acceptable carriers or diluents, the proportion and nature of which are determined by the solubility and chemical properties of the compound selected, the chosen route of administration, and standard pharmaceutical practice. The compounds may be adminstered orally in solid dosage forms, e.g. capsules, tablets, powders, or in liquid forms, e.g. solutions or suspensions. The compound may also be injected parenterally in the form of sterile solutions or suspensions. Solid oral forms may contain conventional excipients, for instance: lactose, succrose, magnesium stearate, resins, and like materials. Liquid oral forms may contain various flavoring, coloring, preserving, stabilizing, solubilizing, or suspending agents. Parenteral preparations are sterile aqueous or nonaqueous solutions or suspensions which may contain various preserving, stabilizing, buffering, solubilizing, or suspending agents. If desired, additives, such as saline or glucose, may be added to make the solutions isotonic.

The amount of active compound administered will vary and can be any effective amount. Unit doses of these compounds can contain, for example, from about 1 mg to 100 mg of the compounds and may be administered, for example, one or more times daily, as needed.

The term "unit dosage form" is used herein to mean a single or multiple dose form containing a quantity of the active ingredient in admixture or otherwise in association with the diluent or carrier, said quantity being such that one or more predetermined units are normally required for a single therapeutic administration. In the case of multiple dose forms, such as liquids or scored tablets, said predetermined unit will be one fraction such as 5 ml (teaspoon) quantity of a liquid or a half or quarter of a scored tablet, of the multiple dose form.

In a composition aspect, the invention provides pharmaceutical compositions comprising a compound of the formula:

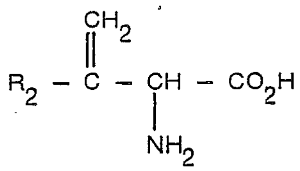 ,
XXVI

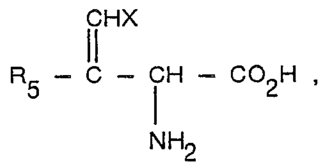 ,
XXVII

or

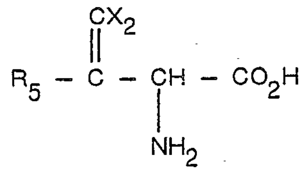
XXVIII

wherein:

X is fluorine, chlorine, or bromine, and

$R_5$ is a group of the formula:

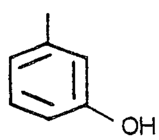 , 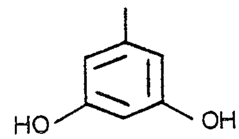 ,

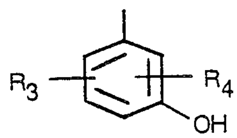 , 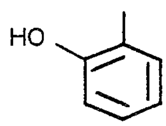 , 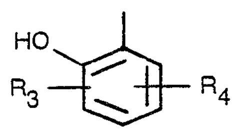 ,

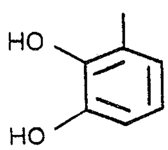 , 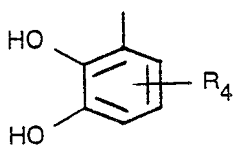 , 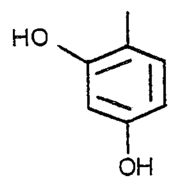 ,

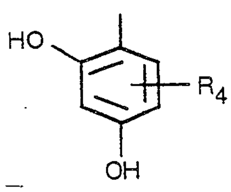 , 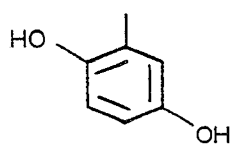 , 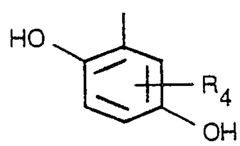 ,

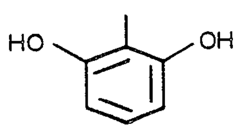 , 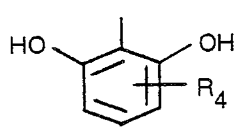 , 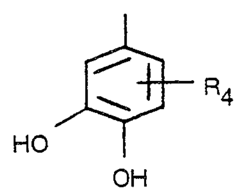 ,

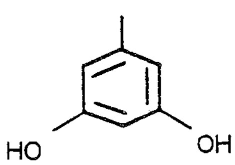 , or 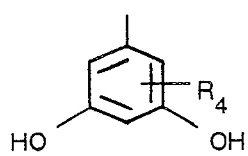 ;

wherein

$R_3$ is hydrogen, $(C_1—C_4)$alkyl, or $(C_1—C_4)$alkoxy, and

$R_4$ is $(C_1—C_4)$alkyl or $(C_1—C_4)$alkoxy;

or a $(C_1—C_8)$alkyl ester thereof; or a non-toxic pharmaceutically acceptable salt thereof; in admixture with or otherwise in association with a pharmaceutically acceptable carrier or diluent thereof.

In another composition aspect, there is provided a pharmaceutical composition comprising (a) a compound of Formula XXVI, XXVII, or XXVIII, wherein X and R are as defined hereinabove, and (b) an aromatic-L-amino acid decarboxylase inhibitor. Both active ingredients can be in admixture with or otherwise associated with a pharmaceutically acceptable carrier or diluent therefor. Preferred aromatic-L-amino acid decarboxylase inhibitors are benzerazide, carbidopa, and a 2-amino-3-(monohydroxyphenyl or dihydroxyphenyl)propionic acid.

The pharmaceutical formulations are prepared in a manner well known *per se* in the pharmaceutical art. The carrier or diluent may be solid, semi-solid, or liquid material which serves as a vehicle, excipient, or medium for the active ingredient. Suitable diluents or carriers are well known *per se*. The pharmaceutical formulations may be adapted for enteral or parenteral use and may be administered to the patient in the form of tablets, capsules, suppositories, solutions, suspensions, or the like.

In the specific examples included hereinbelow, illustrative examples of suitable pharmaceutical formulations are described.

The following examples are illustrative of the processes of the invention. All temperatures are in centigrade.

Example 1

α-Fluoro-3-methoxyacetophenone

A solution of 3-methoxyacetophenone (6.0 g) in acetic acid (20 ml) is treated slowly with bromine (6.4 g) so that the temperature does not rise above 20°. After the completion of the addition, the solution is stirred for 1 hour at 20°, and the acetic acid is completely evaporated. The residue is crystallized from ethanol to give α-bromo-3-methoxyacetophenone (7.51 g) as cream needles.

A mixture of α-bromo-3-methoxyacetophenone (6.87 g) and potassium hydrogen fluoride (7.0 g) in diethylene glycol (50 ml) is heated (100°) and stirred for 5 hours. The mixture is cooled, poured into ice/water, and extracted with ether. The ether extract is washed consecutively with saturated aqueous sodium bicarbonate and water, dried, and evaporated to yield a solid mass. This is recrystallized from a mixture of ethanol and ether to afford pure α-fluoro-3-methoxyacetophenone (3.28 g): colorless needles, m.p. 53—54°:

*NMR* ($CDCl_3$): δ 3.82, s, 3H; 5.43, d (J=46Hz), 2H; 6.93 to 7.53, m, 4H.

*Analysis for* $C_9H_9FO_2$:

Found: C, 64.59; H, 5.34%

Requires: C, 64.28; H, 5.39%

Example 2

Ethyl 3-(3'-methoxyphenyl)-4-fluoro-2-butenoate

A solution of diethyl ethoxycarbonylmethanephosphonate (5.6 g) in dimethoxyethane (80 ml) is added dropwise to a cooled (0°) suspension of sodium hydride (0.6 g) in dimethoxyethane (30 ml). The mixture is stirred at room temperature for 2 hours, and a solution of α-fluoro-3-methoxyacetophenone (4.20 g) in dimethoxyethane (100 ml) is added slowly. Stirring is continued for 30 minutes after the completion of the addition. The solution is cooled to about 10° and then poured into ice-water (200 ml) after which the mixture is extracted with ether. The ether solution is washed with water, dried, and evaporated to yield an orange oil (5.23 g). Purification is achieved by chromatography on silica gel (200 g) using light petroleum (90%)/ether (10%) as eluant whereupon there is obtained pure ethyl 3-(3'-methoxyphenyl)-4-fluoro-2-butenoate (3.92 g): almost colorless oil:

*NMR* ($CDCl_3$): δ 1.28, t (J=7Hz), 3H; 3.77, s, 3H; 4.20, q (J=7Hz); 5.83, d.d (J=47Hz, 1.5Hz); 6.15, s (broad), 1H; 6.73 to 7.43m, m, 4H.

*Analysis for* $C_{13}H_{15}FO_3$:

Found: C: 65.53; H, 6.44%

Requires: C, 65.53; H, 6.35%

Example 3

Ethyl 2,3-dibromo-3-(3'-methoxyphenyl)-4-fluorobutyrate

To a solution of ethyl 3-(3'-methoxyphenyl)-4-fluoro-2-butenoate (1.85 g) in carbon tetrachloride (40 ml) cooled to about −10° is added dropwise a solution of bromine (1.48 g) in carbon tetrachloride (10 ml). Stirring is continued at this temperature for 2 hours, after which the solution is evaporated to dryness. The residue is a pale orange oil (3.27 g) and is essentially pure ethyl 2,3-dibromo-3-(3'-methoxyphenyl)-4-fluorobutyrate:

*NMR* ($CDCl_3$): δ 1.23, t (J=7Hz), 3H; 3.75, s, 3H; 4.20, q (J=7Hz), 2H; 5.00, s, 1H; 5.35, d (J=50Hz), 2H; 6.63 to 7.43, m, 4H.

Example 4

Ethyl 2-bromo-3-(3'-methoxyphenyl)-4-fluoro-2-butenoate

A solution of ethyl 2,3-dibromo-3-(3'-methoxyphenyl)-4-fluorobutyrate (5.55 g) in tetrahydrofuran (10 ml) is added dropwise to a suspension of NaH (0.41 g) in tetrahydrofuran (40 ml). The mixture is refluxed for 5 hours, cooled, and carefully treated with water. The mixture is then extracted with ether. The ether

extract is washed with water, dried, and evaporated to leave an orange oil (4.52 g). Purification is achieved by silica gel (200 g) chromatography using as eluant a mixture of light petroleum (90%)/ether (10%) whereupon there is obtained pure ethyl 2-bromo-3-(3'-methoxyphenyl)-4-fluoro-2-butenoate (3.85 g) almost colorless oil:

*NMR* ($CDCl_3$): δ 0.97, t (J=7Hz), 3H; 3.78, s, 3H; 4.00, q (J=7Hz), 2H; 5.35, d (J=48 Hz), 2H; 6.70 to 7.52, m, 4H.

*Analysis for* $C_{13}H_{14}BrFO_3$:

Found: C: 49.18; H, 4.35%
Requires: C, 49.23; H, 4.45%

Example 5

Ethyl 2-bromo-3-(3'-methoxyphenyl)-4-fluoro-3-butenoate

A solution of ethyl 2-bromo-3-(3'-methoxyphenyl)-4-fluoro-2-butenoate (3.80 g) in tetrahydrofuran (15 ml) is added to a cooled (−75°) solution of lithium diisopropylamide prepared in the normal way from diisopropylamine (2.1 ml) and *n*-butyllithium (10 ml of a 1.5 M solution in hexane) in tetrahydrofuran (60 ml). After the addition, the reaction mixture is stirred for 90 minutes at −75°. Then 5% aqueous hydrochloric acid (10 ml), followed by water (20 ml), are added cautiously. The mixture is extracted with ether, and the ether extract is washed with dilute aqueous hydrochloric acid, then with water, dried, and evaporated to give ethyl 2-bromo-3-(3'-methoxyphenyl)-4-fluoro-3-butenoate (3.76 g) as a brown oil, and can be used without purification.

Example 6

Ethyl 2-*tert*-butoxycarbonylamino-3-(3'-methoxyphenyl)-4-fluoro-3-butenoate

A cooled (5°) saturated solution of ammonia in dimethyl sulfoxide (15 ml) is added to ethyl 2-bromo-3-(3'-methoxyphenyl)-4-fluoro-3-butenoate (0.62 g) in a pressure vessel. The vessel is sealed and allowed to stand for 2 days at room temperature. The solution is then poured into ice-water and extracted with dichloromethane. The dichloromethane extract is washed thoroughly with water, dried, and evaporated to leave an orange oil (0.36 g). This substance is dissolved in tetrahydrofuran (30 ml) containing di-*tert*-butyl dicarbonate (0.31 g) and then stirred at 60° for 2 hours. Evaporation of the solvent and purification of the residue (0.64 g) by silica gel (10 g) chromatography using light petroleum (90%)/ether (10%) as eluant allows the isolation of pure racemic ethyl 2-*tert*-butoxycarbonylamino-3-(3'-methoxyphenyl)-4-fluoro-3-butenoate (0.22 g): colorless oil:

*NMR* ($CDCl_3$): δ 1.17, t (J=7Hz), 3H; 1.42, s, 9H; 3.72, s, 3H; 4.13, q (J=7Hz), 2H; 4.90, d (J=8Hz), 1H; 5.30, d (broad, J=8Hz), 1H; 6.85, d (J=82Hz), 1H; 6.70 to 7.40, m, 4H.

*Analysis for* $C_{18}H_{24}FNO_5$:

Found: C: 61.22; H, 6.66; N, 3.87%
Requires: C, 61.18; H, 6.85; N, 3.96%

Example 7

Ethyl 2-amino-3-(3'-methoxyphenyl)-4-fluoro-3-butanoate

A solution of ethyl 2-*tert*-butoxycarbonylamino-3-(3'-methoxyphenyl)-4-fluoro-3-butenoate (218 mg) in ether (10 ml) saturated with dry HCl is left overnight by which time colorless needles crystallize. These are collected and dried to afford pure racemic ethyl 2-amino-3-(3'-methoxyphenyl)-4-fluoro-3-butenoate (150 mg): colorless needles, m.p. 141—142°:

*Analysis for* $C_{13}H_{17}ClFNO_3$:

Found: C: 54.22; H, 6.25; N, 4.67%
Requires: C, 53.89; H, 5.91; N, 4.83%

Example 8

2-Amino-3-(3'-hydroxyphenyl)-4-fluoro-3-butenoic acid

A solution of ethyl 2-amino-3-(3'-methoxyphenyl)-4-fluoro-3-butenoate (150 mg) in 47% aqueous hydrobromic acid (10 ml) is refluxed for 3 hours after which the water is completely evaporated. The residue (orange oil, 112 mg) is dissolved in ethanol (1 ml) and treated with a slight excess of propylene oxide so that the pH is adjusted to about 4 to 5. The resulting precipitate is collected and subsequently recrystallized from ethanol/water whereupon pure racemic 2-amino-3-(3'-hydroxyphenyl)-4-fluoro-3-butenoic acid (29 mg) is obtained: colorless needles, m.p. 215°:

*NMR* ($CD_3OD$): δ 4.62, s, 1H; 6.28 to 7.13, m, 4H; 6.98, d (J=80Hz), 1H.

*Analysis for* $C_{10}H_{10}FNO_3$:

Found: C: 56.73; H, 5.21; N, 6.49%
Requires: C, 56.87; H, 4.77; N, 6.63%

Example 9

Ethyl 2-bromo-3-(3'-methoxyphenyl)-4-fluoro-2-butenoate

Piperidine (0.22 ml) is added to a solution of ethyl 2,3-dibromo-3-(3'-methoxyphenyl)-4-fluorobutyrate (0.80 g) in ether (10 ml). The reaction mixture is stirred at room temperature for 3 hours and water is added. Ether extraction gives essentially pure ethyl 2-bromo-3-(3'-methoxyphenyl)-4-fluoro-2-butenoate (0.65 g).

Example 10

2-Amino-3-(3'-hydroxy-4'-methoxyphenyl)-4-fluoro-3-butenoic acid

Repeating the procedures described in Examples 2, 3, 9, 5, and 6, α-fluoro-3-tetrahydropyranyloxy-4-methoxyacetophenone can be converted to ethyl 2-*tert*-butoxycarbonylamino-3-(3'-tetrahydropyranyloxy-4-methoxyphenyl)-4-fluoro-3-butenoate. The conversion of this intermediate to the final product is achieved as follows:

A mixture of this product (0.45 g) and lithium hydroxide monohydrate (0.09 g) in water (3 ml) and THF (13 ml) is stirred at room temperature for 2 hours. Dilute aqueous hydrochloric acid is added to adjust the pH to about 4, then the acid is isolated by ether extraction. The crude material is treated overnight with a saturated solution of hydrogen chloride in ether (20 ml). Filtration affords a colorless powder (0.21 g) which is dissolved in ethanol (20 ml) and treated with propylene oxide (0.20 g) at room temperature overnight. Filtration of the precipitate affords 2-amino-3-(3'-hydroxy-4'-methoxyphenyl)-4-fluoro-3-butenoic acid (0.12 g) as a colorless powder, m.p. 214°:

*NMR* ($D_2O$ + DCl): δ 3.87, s, 3H; 5.00, s, 1H; 6.77 to 7.13, m, 3H; 7.25, d (J=80 Hz), 1H.

*Analysis for* $C_{11}H_{12}FNO_4$:

Found: C: 54.68; H, 5.18; N, 5.74%

Requires: C, 54.77; H, 5.01; N, 5.81%

Example 11

α-Fluoro-3-tetrahydropyranyloxy-4-methoxyacetophenone

A solution of 3-acetoxy-4-methoxyacetophenone (53 g) in chloroform (700 ml) is cooled in an ice bath and treated slowly with a solution of bromine (41 g) in chloroform (100 ml). The solution is then stirred for 90 minutes after which the chloroform is evaporated. The residue is dissolved in ether, washed in water, dried and evaporated. The solid residue is recrystallized from dichloromethane/hexane to afford α-bromo-3-acetoxy-4-methoxyacetophenone (59.51 g) as colorless plates: m.p. 116—117°.

A portion (57.4 g) of this bromide, mixed with $KH_2F$ (46.8 g) in diethylene glycol (500 ml), is allowed to remain at 100° for 8 hours. The mixture is cooled, poured into ice-water, and extracted with ether. The ether soluble material is purified by silica gel chromatography using light petroleum (50%)/diethyl ether (50%) as eluant whereupon α-fluoro-3-hydroxy-4-methoxyacetophenone (22.4 g) is obtained. Recrystallization from dichloromethane/hexane affords colorless plates (20.2 g): m.p. 70—71°.

The phenol (18.35 g) is treated with dihydropyran (20 ml) and concentrated hydrochloric acid (10 drops) at room temperature for 3½ hours. The solution is poured into 2% aqueous sodium hydroxide (300 ml) and extracted with ether. The ether soluble material is purified by silica gel chromatography (light petroleum (80%)/diethyl ether (20%)), and by recrystallization from dichloromethane/hexane to afford α-fluoro-3-tetrahydropyranyloxy-4-methoxyacetophenone (17.15 g), as colorless needles: m.p. 78—79°:

*NMR* ($CDCl_3$): δ 1.57 to 1.93, m, 6H; 3.43 to 4.10, m and 3.87, s, 5H; 5.40, d (J=47 Hz), 2H; 5.40, s (broad), 1H; 6.80 to 7.63, ABC system, 3H.

*Analysis for* $C_{14}H_{17}FO_4$:

Found: C: 62.59; H, 6.32%

Requires: C, 62.68; H, 6.39%

Example 12

Ethyl 3-(3'-tetrahydropyranyloxyphenyl)-2-butenoate

A solution of diethyl ethoxycarbonylmethanephosphonate (10.3 g) in dimethoxyethane (100 ml) is added dropwise to a cooled (0°) suspension of sodium hydride (1.2 g) in dimethoxyethane (50 ml). When the addition is complete (about 30 minutes), the cooling bath is removed and the mixture is stirred for a further 2 hours. To this mixture is slowly added a solution of 3-tetrahydropyranyloxyphenylacetophenone (10.0 g) in dimethoxyethane (200 ml) at room temperature. The reaction mixture is then heated at about 70° for 4 hours, cooled to room temperature, and poured into water (1 l). Ether extraction followed by silica gel (200 g) chromatography (light petroleum (80%)/ether (20%) as eluant) enables the isolation of pure ethyl 3-(3'-tetrahydropyranyloxyphenyl)-2-butenoate (8.45 g): colorless oil:

*NMR* ($CDCl_3$): δ 1.27, t (J=7Hz), 3H; 1.40 to 2.07, m, 6H; 2.50, d (J=1.5 Hz), 3H; 3.33 to 3.93, m, 2H; 4.13, q (J=7Hz), 2H; 5.35, s (broad), 1H; 4.37, t (J=1.5 Hz), 1H; 6.83 to 7.57, m, 4H.

Example 13

*Step A:*

Ethyl 2,3-dibromo-3-(3'-tetrahydropyranyloxyphenyl)butyrate

A solution of bromine (3.50 g) in carbon tetrachloride (60 ml) is added dropwise to a cooled (−10°) solution of ethyl 3-(3'-tetrahydropyranyloxyphenyl)-2-butenoate (5.80 g) and pyridine (10 drops) in carbon

tetrachloride (60 ml). After the completion of the addition, the reaction mixture is stirred for 1 hour at about −10°, and the solvent is evaporated to leave an orange oil (9.42 g). Chromatography on silica gel (200 g) using a mixture of light petroleum (80%) and ether (20%) as eluant allows the separation of pure ethyl 2,3-dibromo-3-(3′-tetrahydropyranyloxyphenyl)butyrate (5.47 g): colorless oil:

*NMR* ($CDCl_3$): δ 1.37, t (J=7Hz), 3H; 1.27 to 2.20, m, 6H; 2.55, s, 3H; 3.40 to 4.13, m, 2H; 4.33, q (J=7Hz), 2H; 5.05, s, 1H; 5.10, s (broad), 1H; 6.73 to 7.43, m, 4H.

*Step B:*
(Racemic) Ethyl 2-(tert-butoxycarbonylamino)-3-(3′-tetrahydropyranyloxyphenyl)-3-butenoate

A saturated solution of ammonia in dimethyl sulfoxide (20 ml) is added to ethyl 2,3-dibromo-3-(3′-tetrahydropyranyloxyphenyl)butyrate (1.5 g) in a suitable pressure vessel at about 5°. The reaction vessel is firmly sealed. The solution is left at ambient temperature for 40 hours and is then poured into ice-cold water (200 ml) which is extracted with dichloromethane. The organic extract is thoroughly washed with water, dried, and evaporated to leave a yellow oil (0.64 g).

A solution of this material and di-*tert*-butyl dicarbonate (0.45 g) in tetrahydrofuran (50 ml) is heated at 60° for 2 hours. Evaporation to dryness leaves an orange oil (0.93 g). Purification is achieved by silica gel (30 g) chromatography using light petroleum (90%)/ether (10%) as eluant whereupon there is obtained racemic ethyl 2-(*tert*-butoxycarbonylamino)-3-(3′-tetrahydropyranyloxyphenyl)-3-butenoate (0.43 g): colorless oil:

*NMR* ($CDCl_3$): δ 1.08, t (J=7Hz), 3H; 1.37, s, 9H; 1.40 to 2.10, m, 6H; 3.30 to 3.90, m, 2H; 4.08, q (J=7Hz), 2H; 4.92 to 5.53, m, 5H; 6.77 to 7.33, m, 4H.

*Step C:*
(Racemic) 2-(tert-Butoxycarbonylamino)-3-(3′-tetrahydropyranyloxyphenyl)-3-butenoic acid

A solution of ethyl 2-(*tert*-butoxycarbonylamino)-3-(3′-tetrahydropyranyloxyphenyl)-3-butenoate (0.40 g) in dimethoxyethane (10 ml) and water (2 ml) is treated with solid lithium hydroxide monohydrate (0.02 g). After stirring for 2 hours at room temperature, the mixture is diluted with water and acidified with 0.1 N hydrochloric acid to *ca* pH 4. Extraction with ether gives essentially pure racemic 2-(*tert*-butoxycarbonylamino)-3-(3′-tetrahydropyranyloxyphenyl)-3-butenoic acid (0.25 g): colorless solid.

*Step D:*
Hydrochloride of (racemic) 2-amino-3-(3′-hydroxyphenyl)-3-butenoic acid

A solution of 2-(*tert*-butoxycarbonylamino)-3-(3′-tetrahydropyranyloxyphenyl)-3-butenoic acid (0.25 g) in ether (10 ml) saturated with dry hydrogen chloride is left standing at about 5° for 16 hours during which time colorless crystals precipitate. These are collected and dried to give the hydrochloride of racemic 2-amino-3-(3′-hydroxyphenyl)-3-butenoic acid (0.12 g): colorless needles, m.p. 199—200°:

*Analysis for* $C_{10}H_{12}ClNO_3$:

| | | | |
|---|---|---|---|
| Found: | C, 52.08; | H, 5.24; | N, 5.95% |
| Requires: | C, 52.29; | H, 5.27; | N, 6.10% |

*Step E:*
(Racemic) 2-Amino-3-(3′-hydroxyphenyl)-3-butenoic acid

A solution of the hydrochloride of 2-amino-3-(3′-hydroxyphenyl)-3-butenoic acid (0.12 g) in ethanol (2 ml) is treated with a slight excess of propylene oxide. After several hours, the precipitated material is collected and dried to afford pure racemic 2-amino-3-(3′-hydroxyphenyl-3-butenoic acid: colorless powder, m.p. 204—205°:

*NMR* ($CD_3OD$): δ 4.8, s obscured by $CD_3$ OH peak; 5.33, s, 1H; 5.47, s, 1H; 6.47 to 7.13, m, 4H.

In the following Examples relating to pharmaceutical compositions, the term "active compound" is used to indicate the compound 2-amino-3-(3′-hydroxy)phenyl-3-fluoroallylamine. This compound may be replaced in these compositions by any other compound of the invention. Adjustments in the amount of medicament may be necessary or desirable depending upon the degree of activity of the medicament as is well known in the art.

Example 14

An illustrative composition for hard gelatin capsules is as follows:

| | |
|---|---|
| (a) active compound | 0.5 mg |
| (b) talc | 5 mg |
| (c) lactose | 94.5 mg |

The formulation is prepared by passing the dry powders of (a) and (b) through a fine mesh screen and mixing them well. The powder is then filled into hard gelatin capsules at a net fill of 100 mg per capsule.

Example 15

An illustrative composition for tablets is as follows:

| | |
|---|---|
| (a) active compound | 0.5 mg |
| (b) starch | 48 mg |
| (c) lactose | 51.5 mg |
| (d) magnesium stearate | 2 mg |

The granulation obtained upon mixing the lactose with the compound (a) and the part of the starch and granulated with starch paste is dried, screened, and mixed with the magnesium stearate. The mixture is compressed into tablets weighing 100 mg each.

Example 16

An illustrative composition for an injectable suspension is the following 1 ml ampul for an intramuscular injection:

| | weight per cent |
|---|---|
| (a) active compound | 0.1 |
| (b) polyvinylpyrrolidone | 0.5 |
| (c) lecithin | 0.25 |
| (d) water for injection to make | 100 |

The materials (a)—(d) are mixed, homogenized, and filled into 1 ml ampuls which are sealed and autoclaved 20 minutes at 121°. Each ampul contains 1 mg per ml of active compound.

Example 17

| | mg/suppository |
|---|---|
| Active compound | 1 |
| Oil of Theobroma | 999 |

The medicament is powdered and passed through a B.S. No. 100 sieve and triturated with molten oil of Theobroma at 45° to form a smooth suspension. The mixture is well stirred and poured into moulds each of nominal 1 G capacity, to produce suppositories.

Example 18

2-Amino-3-(3'-hydroxyphenyl)-3-butenoic acid (hereinafter referred to as "AHBA"), 2-amino-4-fluoro-3-(3'-hydroxyphenyl)-3-butenoic acid (hereinafter referred to as "AFHBA"), and 2-amino-4-fluoro-3-(3'-hydroxy-4-methoxyphenyl)-3-butenoic acid (hereinafter referred to as "AFMBA") were tested as follows:

A. *In vitro* testing

AHBA or AFHBA was incubated with partially purified hog kidney AADC at 37° for various times up to 2 hours. HPLC analysis determined that at 2 hours each compound (as the DL-mixture) underwent 50% decarboxylation to give the corresponding allylamine [2-(3'-hydroxy)phenylallylamine or 2-(3'-hydroxy)phenyl-3-fluoroallylamine, respectively]. When the experiment was repeated in the presence of 10 μM α-monofluoromethyl-Dopa (MFMD) (an AADC inhibitor), no decarboxylation was observed.

The decarboxylated products are time-dependent irreversible inhibitors of MAO *in vitro*: $IC_{50}$ for 2-(3'-hydroxy)phenylallylamine, $\sim10^{-5}$; $IC_{50}$ for 2-(3'-hydroxy)phenyl-3-fluoroallylamine, $\sim10^{-9}$. AHBA and AFHBA are inactive or very weak inhibitors of MAO.

B. Ex vivo testing

I. Mice were injected with either AHBA (250 mg/kg, i.p.) alone or with AHBA (250 mg/kg, i.p.) in combination with MFMD (1 mg/kg, i.p.) adminstered 30 minutes before AHBA. Mice were killed 4 hours later and MAO activity (tyramine as substrate) was determined in brain and heart. AHBA administered alone produced 30% inhibition of MAO in brain and heart. AHBA in combination with MFMD produced 65% inhibition of MAO in the brain.

To determine the site-selective effect, MAO activity was determined in mouse brain using the neuronal substrate 5—HT and the non-neuronal substrate phenylethylamine (PEA). AHBA administered in

combination with MFMD inhibited neuronal MAO (5—HT substrate) by 82% and non-neuronal MAO (PEA substrate) by 15%.

II. Rats were injected with AFHBA (0.5 mg/kg, i.p.) alone or with AFHBA (0.5 mg/kg, i.p.) in combination with MFMD (2.0 mg/kg, i.p.) administered 30 minutes before AFHBA. Animals were killed 18 hours later and MAO activity (5—HT and phenethylamine as substrates) was determined in brain, heart, and liver. In the brain, AFHBA administered alone inhibited neuronal MAO by 72% and non-neuronal MAO by 37%. Pretreatment with MFMD did not essentially reduce the inhibition of neuronal MAO (68%), but it reduced inhibition of non-neuronal MAO to 28%. In the heart, AFHBA inhibited neuronal MAO by 52% and non-neuronal MAO by 44%, but pretreatment with MFMD reduced neuronal inhibition of MAO to 18% and non-neuronal inhibition to 4%. In the liver, MAO inhibition by AFHBA alone was 29% (neuronal) and 38% (non-neuronal), but pretreatment with MFMD totally blocked MAO-inhibition.

When the above experiments with AFHBA were repeated using carbidopa (50 mg/kg, i.p.), the AADC inhibitor produced the same protective effect against inhibition of MAO in the heart as MFMD (2.0 mg/kg, i.p.). To demonstrate the activity of AFHBA by oral administration, rats were gavaged with various doses of the compounds and killed 18 hours later. MAO activity was determined in the brain using 5—HT and phenethylamine (PEA) as substrates. The following results were obtained:

| | % Inhibition of MAO | |
|---|---|---|
| Dose (mg/kg) | Neuronal | Non-neuronal |
| 0.5 | 63 | 41 |
| 1.0 | 71 | 64 |
| 2.5 | 95 | 83 |

III. Rats were injected with AFMBA (100 mg/kg, i.p.) and were killed 18 hours later. MAO activity was determined in the brain, heart, and liver using 5—HT and PEA. The following results were obtained:

| | % Inhibition of MAO | |
|---|---|---|
| Dose (mg/kg) | Neuronal | Non-neuronal |
| Brain | 16 | 40 |
| Heart | 18 | 46 |
| Liver | 2 | 11 |

IV. Rats were injected with AHBA as the ethyl ester (334 mg/kg, i.p.) alone or in combination with carbidopa (100 mg/kg, i.p.) given 30 minutes before administration of the ethyl ester of AHBA. The animals were killed 4 hours later. MAO activity was determined in the brain using 5—HT and PEA as substrates. The following results were obtained:

| | % Inhibition of MAO | | | | | |
|---|---|---|---|---|---|---|
| | Brain | | Heart | | Liver | |
| Treatment | N* | Non-N* | N* | Non-N* | N* | Non-N* |
| AHBA, ethyl ester | 59 | 40 | 23 | 3 | 37 | 0 |
| AHBA, ethyl ester + carbidopa | 78 | 31 | 0 | 0 | 20 | 13 |

N* means Neuronal.

C. *In vivo* testing

Rats were injected with AFHBA (0.5 mg/kg, i.p.) alone or with AFHBA (0.5 mg/kg, i.p.) in combination with MFMD (2 mg/kg, i.p.) or carbidopa (50 mg/kg, i.p.) given 30 minutes before the AFHBA. The rats were killed 18 hours later, and dopamine, DOPAC, 5—HT, and 5—HIAA concentrations were determined in the brain. The following results were obtained:

| Treatment | % Change from Control | | | |
|---|---|---|---|---|
| | Dopamine | DOPAC | 5—HT | 5—HIAA |
| AFHBA | +25 | −76 | +30 | −34 |
| AFHBA + MFMD | +50 | −92 | +92 | −48 |
| AFHBA + carbidopa | +54 | −89 | +92 | −37 |

In a separate experiment, rats were given AFHBA orally at various doses. The rats were killed 18 hours later and dopamine, DOPAC, 5—HT, and 5—HIAA concentrations were determined in the brain. The following results were obtained:

| Dose (mg/kg) | % Change from Control | | | |
|---|---|---|---|---|
| | Dopamine | DOPAC | 5—HT | 5—HIAA |
| 0.5 | + 8 | −55 | + 26 | −11 |
| 1.0 | +17 | −72 | + 74 | −23 |
| 2.5 | +28 | −79 | +140 | −41 |

**Claims for the Contracting States: BE CH DE FR IT LI LU NL SE**

1. A compound of the formula:

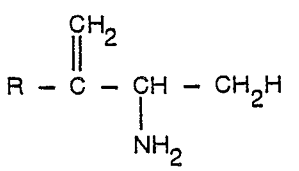
,
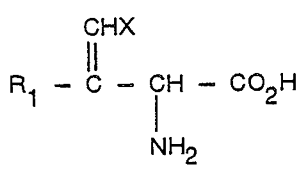
, or
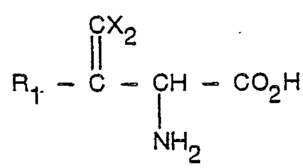

I II III

wherein:

X is fluorine, chrone, or bromine;

R is the group $R_2$ as defined below;

$R_1$ is a group of the formula:

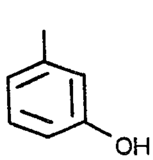
or
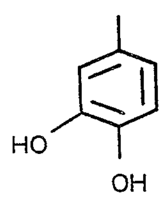

or the group $R_2$ as defined below;

wherein $R_2$ is:

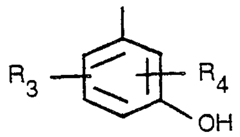 , 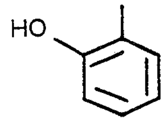 , 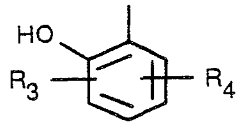 ,

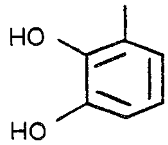 , 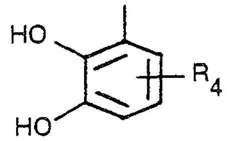 , 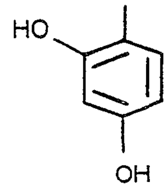 ,

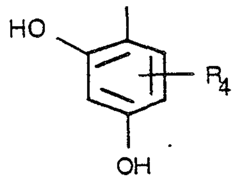 , 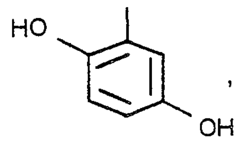 , 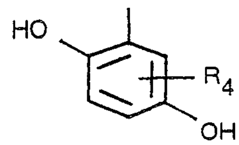 ,

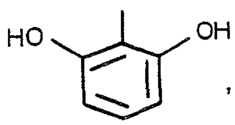 , 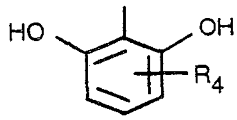 , 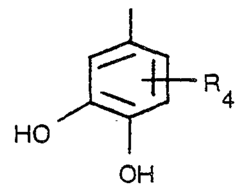 ,

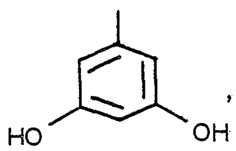 , or 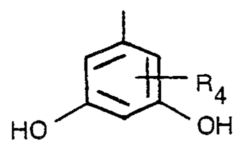 ;

wherein $R_3$ is hydrogen, $(C_1—C_4)$alkyl, or $(C_1—C_4)$alkoxy, and $R_4$ is $(C_1—C_4)$alkyl or $(C_1—C_4)$alkoxy;
or an non-toxic pharmaceutically acceptable salt thereof.

2. A compound of Formula I as claimed in Claim 1.

3. A compound of Formula II as claimed in Claim 1.

4. A compound of Formula III as claimed in Claim 1.

5. A compound as claimed in any one of Claims 3 and 4 wherein $R_1$ is:

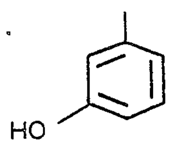 .

6. A compound as claimed in any one of Claim 3 and 4 wherein $R_1$ is:

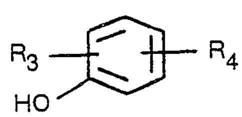 ,

wherein $R_3$ is hydrogen, $(C_1—C_4)$, or $(C_1—C_4)$alkoxy, and $R_4$ is $(C_1—C_4)$ alkyl or $(C_1—C_4)$alkoxy.

7. A compound as claimed in Claim 6 wherein $R_3$ is hydrogen.

8. A compound as claimed in any one of Claims 3 to 7 wherein X is fluorine.

9. 2-Amino-4-fluoro-3-(3′-hydroxyphenyl)-3-butenoic acid or a non-toxic pharmaceutically acceptable salt thereof.

10. A compound as claimed in any one of the preceding claims for use in the inhibition in the human body of monoamine oxidase.

11. A compound as claimed in any one of the preceding claims for use in the therapy of depression in the human body

12. A pharmaceutical composition comprising a compound in any one of the preceding claims in admixture or otherwise associated with a pharmaceutically acceptable carrier or diluent therefor.

13. A pharmaceutical composition as claimed in Claim 12 in unit dosage form containing 1 mg to 100 mg of said compound per unit dose.

14. A pharmaceutical composition comprising (a) a compound as claimed in any one of Claims 1 to 11 and (b) an aromatic L-amino acid decarboxylase inhibitor.

15. A pharmaceutical composition as claimed in Claim 14 wherein the active ingredients are in admixture or otherwise associated with a pharmaceutically acceptable carrier or diluent therefor.

16. A Compound selected from 2-amino-3-(3'-hydroxyphenyl)-3-butenoic acid, 2-amino-3-(3',4'-dihydroxyphenyl)-3-butenoic acid, or a non-toxic pharmaceutical acceptable salt thereof for use in the therapy of depression in the human body.

17. A pharmaceutical composition comprising 2-amino-3-(3'-hydroxyphenyl)-3-butenoic acid, or a non-toxic pharmaceutically acceptable salt thereof, in admixture or otherwise associated with a pharmaceutically acceptable carrier or diluent therefor.

18. A pharmaceutical composition comprising 2-amino-3-(3',4'-dihydroxyphenyl)-3-butenoic acid, or a non-toxic pharmaceutically acceptable salt thereof, in admixture or otherwise associated with a pharmaceutically acceptable carrier or diluent therefor.

19. A pharmaceutical composition comprising (a) 2-amino-3-(3'-hydroxyphenyl)-3-butenoic acid, or a non-toxic pharmaceutically acceptable salt thereof, and (b) an aromatic L-amino acid decarboxylase inhibitor.

20. A pharmaceutical composition comprising (a) 2-amino-3-(3'-4'-dihydroxyphenyl)-3-butenoic acid, or a non-toxic pharmaceutically acceptable salt thereof, and (b) an aromatic L-amino acid decarboxylase inhibitor.

21. A pharmaceutical composition as claimed in any one of Claims 19 and 20 wherein the active ingredients are in admixture or otherwise associated with a pharmaceutically acceptable carrier or diluent therefor.

22. A pharmaceutical composition as claimed in any one of Claims 14, 15, 19, 20 and 21 wherein the aromatic L-amino acid decarboxylase inhibitor is carbidopa, benzerazide, or a 2-amino-2-(monofluoromethyl or difluoromethyl)-3-(monohydroxyphenyl or dihydroxyphenyl)propionic acid.

23. A process of preparing a compound as claimed in Claim 1 which comprises hydrolyzing a compound of the formula:

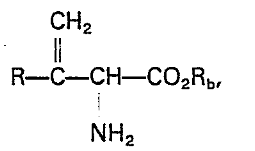 , 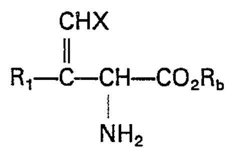 or 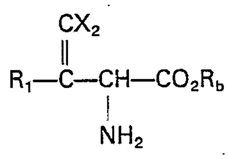

wherein:

R, $R_1$, and X are as defined in Claim 1 and

$R_b$ is $(C_1—C_4)$alkyl;

or an aromatic hydroxy-protected and/or amino-protected derivative thereof; and, if necessary, removing in manner known *per se* the protecting group(s) either simultaneously with or subsequent to the hydrolysis reaction.

**Claims for the Contracting State: AT**

1. A process for preparing a compound of the formula:

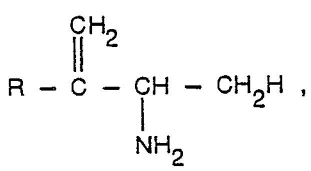 , 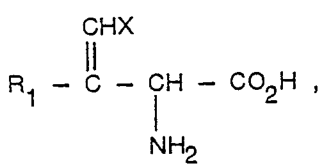 , or 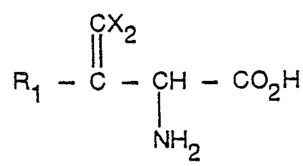

I II III

wherein:

X is fluorine, chlorine, or bromine;

R is the group $R_2$ as defined below;

$R_1$ is a group of the formula:

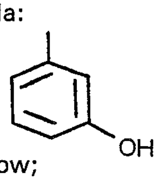 or 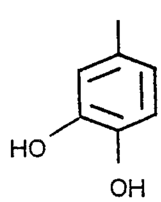

or the group $R_2$ as defined below;

wherein $R_2$ is:

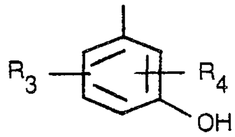 , 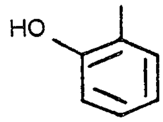 , 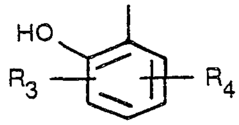 ,

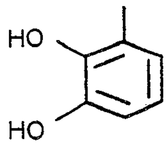 , 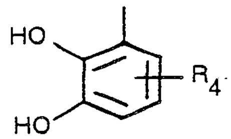 , 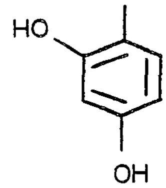 ,

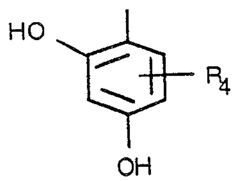 , 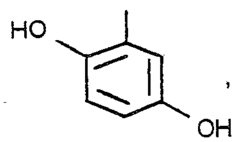 , 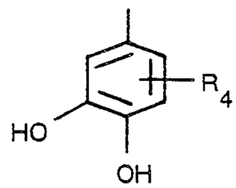 ,

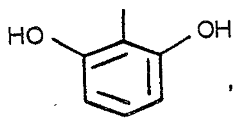 , 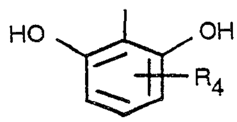 , 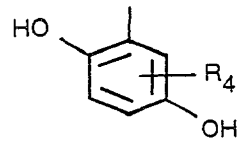 ,

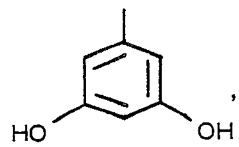 , or 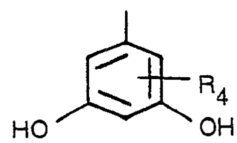 ;

wherein $R_3$ is hydrogen, $(C_1—C_4)$alkyl, or $(C_1—C_4)$alkoxy, and

$R_4$ is $(C_1—C_4)$alkyl or $(C_1—C_4)$alkoxy; or a non-toxic pharmaceutically acceptable salt thereof which comprises hydrolyzing a compound of the formula

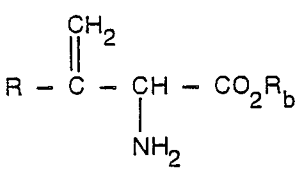 , 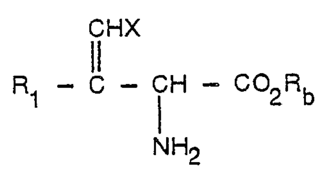 , or 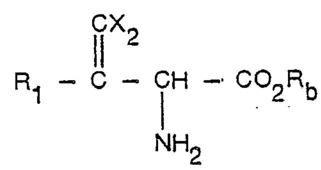

wherein:

R, $R_1$, and X are defined above and

$R_b$ is $(C_1—C_4)$alkyl;

or an aromatic hydroxy-protected and/or amino-protected derivative thereof; and, if necessary, removing in manner known *per se* the protecting group(s) either simultaneously with or subsequent to the hydrolysis reaction.

2. A method as claimed in Claim 1 wherein said compound treated is an aromatic hydroxy-protected derivative.

3. A method as claimed in Claim 1 wherein said compound treated is an amino-protected derivative.

4. A method as claimed in Claim 1 wherein said compound treated is an aromatic hydroxy-protected and amino-protected derivative.

5. A method as claimed in Claim 1 wherein said compound treated is a compound of the formula:

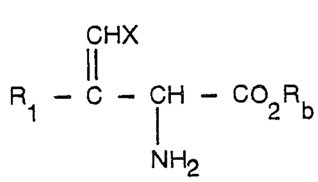 or 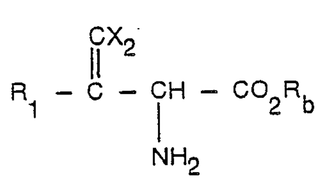

wherein:

$R_b$ is $(C_1—C_4)$alkyl,

X is fluorine, chlorine, or bromine, and

$R_1$ is

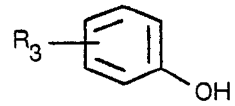

wherein $R_3$ is hydrogen or $(C_1—C_4)$alkyl; or an aromatic hydroxy-protected and/or amino-protected derivative thereof.

6. A method as claimed in Claim 5 wherein said compound treated is a $(C_1—C_4)$alkyl ester of 2-amino-4-fluoro-3-(3'-hydroxyphenyl)-3-butenoic acid.

7. A method as claimed in Claim 5 wherein said compound treated is an aromatic hydroxy-protected derivative of a $(C_1—C_4)$alkyl ester of 2-amino-4-fluoro-3-(3'-hydroxyphenyl)-3-butenoic acid.

8. A method as claimed in Claim 5 wherein said compound treated is an amino-protected derivative of a $(C_1—C_4)$alkyl ester of 2-amino-4-fluoro-3-(3'-hydroxyphenyl)-3-butenoic acid.

9. A method as claimed in Claim 5 wherein said compound is an aromatic hydroxy-protected and an amino-protected derivative of a $(C_1—C_4)$alkyl ester of 2-amino-4-fluoro-3-(3'-hydroxyphenyl)-3-butenoic acid.

10. A method as claimed in any one of Claims 1 to 9 wherein the hydrolysis reaction is carried out using an aqueous strong acid at reflux temperature.

11. A method as claimed in Claim 10 wherein the hydrolysis reaction is carried out using 47% aqueous hydrogen bromide at reflux temperature.

12. A process as in claim 1 for preparing 2-amino-3-(3'-hydroxyphenyl)-3-butenoic acid, 2-amino-3-(3',4'-hydroxyphenyl)-3-butenoic acid, or a non-toxic pharmaceutically acceptable salt thereof for use in the therapy of depression in the human body.

**Patentansprüche für die Vertragsstaaten: BE CH DE FR IT LI LU NL SE**

1. Verbindung der allgemeinen Formel:

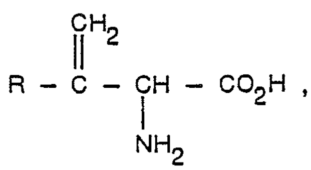 , 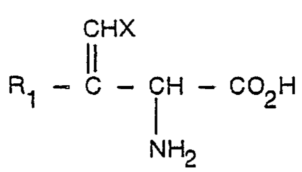 , oder 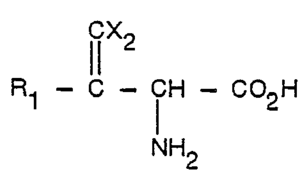

I II III

in der

X ein Fluor-, Chlor- oder Bromatom ist,

R den nachstehend definierten Rest $R_2$ bedeutet; und

$R_1$ eine Gruppe der Formel

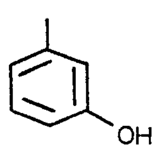 oder 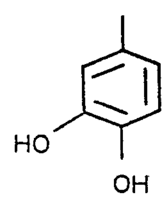

oder den nachstehend definierten Rest $R_2$ bedeutet; wobei $R_2$ die folgende Bedeutung hat:

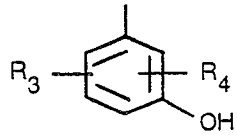 , 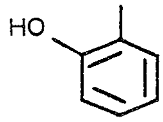 , 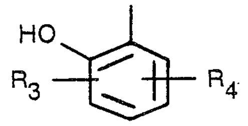 ,

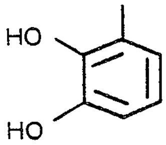 , 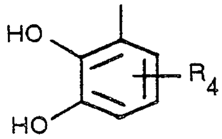 , 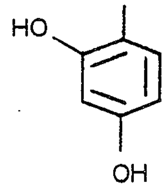 ,

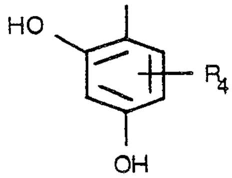 , 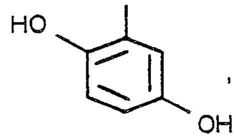 , 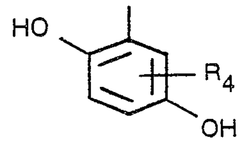 ,

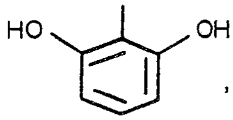 , 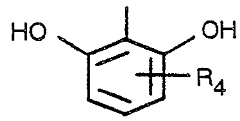 , 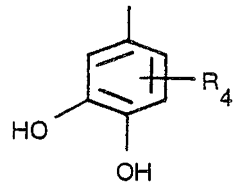 ,

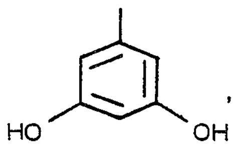 , oder 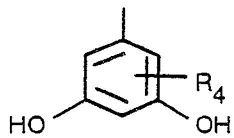 ;

wobei $R_3$ ein Wasserstoffatom, ein $C_1$—$C_4$-Alkylrest oder ein $C_1$—$C_4$-Alkoxyrest ist und $R_4$ einen $C_1$—$C_4$-Alkylrest oder einen $C_1$—$C_4$-Alkoxyrest bedeutet;
oder ein nicht-toxisches pharmakologisch verträgliches Salz davon.

2. Verbindung der allgemienen Formel I nach Anspruch 1.

3. Verbindung der allgemeinen Formel II nach Anspruch 1.

4. Verbindung der allgemeinen Formel III nach Anspruch 1.

5. Verbindung nach einen der Ansprüche 3 oder 4, in dem $R_1$ die folgende Bedeutung hat:

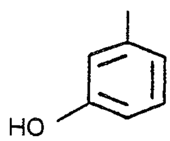

6. Verbindung nach einem der Ansprüche 3 oder 4, in dem $R_1$ die folgende Bedeutung hat:

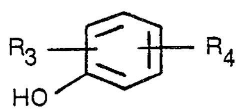 ,

wobei $R_3$ ein Wasserstoffatom, einen $C_1$—$C_4$-Alkyl- oder einen $C_1$—$C_4$-Alkoxyrest bedeutet und $R_4$ ein $C_1$—$C_4$-Alkyl- oder ein $C_1$—$C_4$-Alkoxyrest ist.

7. Verbindung nach Anspruch 6, in der $R_3$ ein Wasserstoffatom ist.

8. Verbindung nach einem der Ansprüche 3 bis 7, in der X ein Fluoratom ist.

9. 2-Amino-4-fluor-3-(3'-hydroxyphenyl)-3-butensäure oder ein nicht-toxisches pharmakologisch verträgliches Salz davon.

10. Verbindung nach einem der vorhergehenden Ansprüche zur Verwendung bei der Hemmung der Monoaminooxidase im menschlichen Körper.

11. Verbindung nach einem der vorhergehenden Ansprüche zur verwendung in der Behandlung von Depressionen beim Menschen.

12. Pharmazeutische Zusammensetzung, enthaltend eine Verbindung nach einem der vorhergehenden Ansprüche in einem Gemisch oder in anderer Weise verbunden mit einem pharmakologisch verträglichen Träger oder einem Verdünnungsmittel.

13. Pharmazeutische Zusammensetzung nach Anspruch 12, in einer Dosierungseinheit, die 1 mg bis 100 mg der vorgenannten Verbindung pro Dosiseinheit enthält.

14. Pharmazeutische Zusammensetzung, enthaltend a) eine Verbindung nach einem der Ansprüche 1 bis 11 und b) einen Inhibitor der Decarboxylase aromatischer L-Aminosäuren.

15. Pharmazeutische Zusammensetzung nach Anspruch 14, in der die Wirkstoffe in einem Gemisch oder in anderer Weise verbunden mit einem pharmakologisch verträglichen Träger oder einem Verdünnungsmittel vorliegen.

16. Verbindung aus der Gruppe 2-Amino-3-(3'-hydroxyphenyl)-3-butensäure, 2-Amino-3-(3',4'-dihydroxyphenyl)-3-butensäure oder ein nicht-toxisches pharmakologisch verträgliches Salz davon zur Verwendung in der Behandlung von Depressionen beim Menschen.

17. Pharmazeutische Zusammensetzung, enthaltend 2-Amino-3-(3'-hydroxyphenyl)-3-butensäure oder ein nicht-toxisches pharmakologisch verträgliches Salz davon im Gemisch oder in anderer Weise verbunden mit einem pharmakologisch verträglichen Träger oder Verdünnungsmittel.

18. Pharmazeutische Zusammensetzung, enthaltend 2-Amino-3-(3',4'-dihydroxyphenyl)-3-butensäure oder ein nicht-toxisches pharmakologisch verträgliches Salz davon im Gemisch oder in anderer Weise verbunden mit einem pharmakologisch verträglichen Träger oder Verdünnungsmittel.

19. Pharmazeutische Zusammenstzung, enthaltend a) 2-Amino-3-(3'-hydroxyphenyl)-3-butensäure oder ein nicht-toxisches pharmakologisch verträgliches Salz davon und b) einen Inhibitor der Decarboxylase aromatischer L-Aminosäuren.

20. Pharmazeutische Zusammensetzung, enthaltend a) 2-Amino-3-(3',4'-dihydroxyphenyl)-3-butensäure oder ein nicht-toxisches pharmakologisch verträgliches Salz davon und b) einen Inhibitor der Decarboxylase aromatischer L-Aminosäuren.

21. Pharmazeutische Zusammensetzung nach einem der Ansprüche 19 oder 20, in der die Wirkstoffe im Gemisch oder in anderer Weise verbunden mit einem pharmakologisch verträglichen Träger oder Verdünnungsmittel vorliegen.

22. Pharmazeutische Zusammensetzung nach einem der Ansprüche 14, 15, 19, 20 oder 21, in der der Inhibitor der Decarboxylase aromatischer L-Aminosäuren Carbidopa, Benzerazid oder 2-Amino-2-(monofluormethyl- oder difluormethyl)-3-(monohydroxyphenyl- oder dihydroxypheny)-propionsäure ist.

23. Verfahren zur Herstellung einer Verbindung nach Anspruch 1, gekennzeichnet durch Hydrolyse einer Verbindung der allgemeinen Formel:

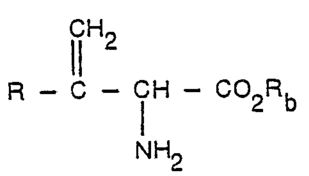 , 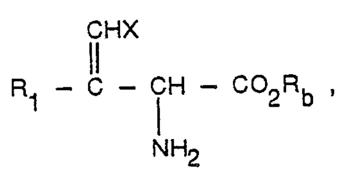 , oder 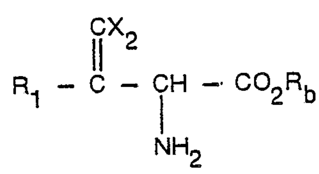

worin

R, $R_1$ und X die gleiche Bedeutung wie in Anspruch 1 haben und

$R_b$ einen $C_1$—$C_4$-Alkylrest bedeutet;

oder eines Derivats davon mit geschützen aromatischen Hydroxyl- und/oder Aminogruppen; und, falls erforderlich, durch die Entfernung der Schutzgruppe(n) in üblicher Weise entweder gleichzeitig mit oder nach der Hydrolysereaktion.

**Patentansprüche für den Vertragsstaat: AT**

1. Verfahren zur Herstellung einer Verbindung der allgemeinen Formel:

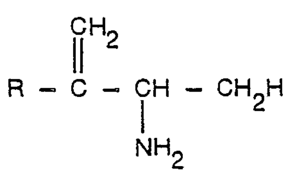 , 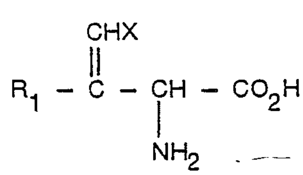 , oder 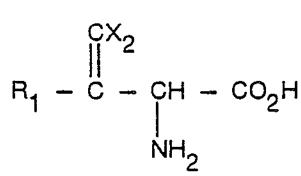

I II III

in der:

X ein Fluor-, Chlor- oder Bromatom ist;

R den nachstehend definierten Rest $R_2$ bedeutet;
$R_1$ eine Gruppe der Formel:

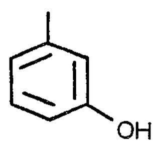 oder 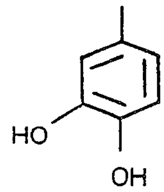

oder den nachstehend definierten Rest $R_2$ bedeutet; wobei $R_2$ die folgende Bedeutung hat:

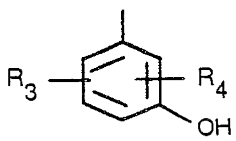, 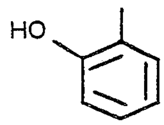, 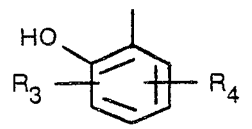,

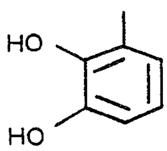, 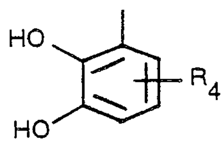, 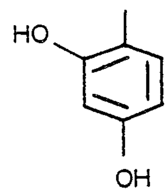,

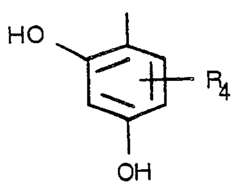, 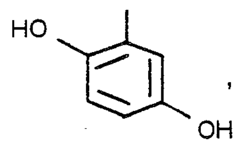, 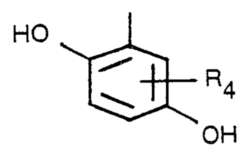,

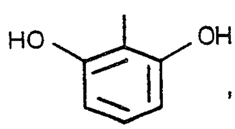, 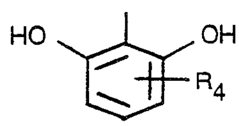, 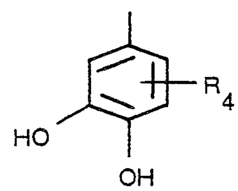,

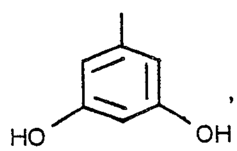, oder 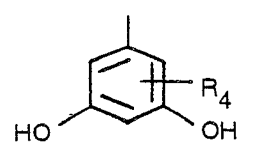;

worin $R_3$ ein Wasserstoffatom, einen $C_1$—$C_4$-Alkylrest oder einen $C_1$—$C_4$-Alkoxyrest und $R_4$ einen $C_1$—$C_4$-Alkylrest oder einen $C_1$—$C_4$-Alkoxyrest bedeutet; oder ein nicht-toxisches pharmakologisch verträgliches Salz davon, gekennzeichnet durch Hydrolyse einer Verbindung der allgemeinen Formel

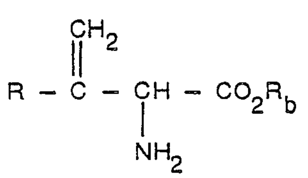, 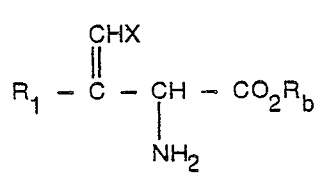, oder 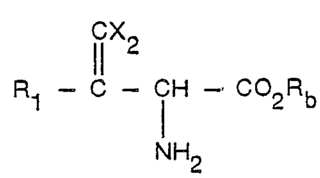

in der:
R, $R_1$ und X die vorstehend angegebenen Bedeutungen haben und
$R_b$ ein $C_1$—$C_4$-Alkylrest ist;

oder eines Derivats davon mit geschützen aromatischen Hydroxyl- und/oder Aminogruppen; und, falls erforderlich, die Entfernung der Schutzgruppe(n) in üblicher Weise entweder gleichzeitig mit oder nach der Hydrolysereaktion.

2. Verfahren nach Anspruch 1, in dem die vorgenannte umgesetzte Verbindung ein Derivat mit geschützten aromatischen Hydroxylgruppen ist.

3. Verfahren nach Anspruch 1, in dem die vorgenannte umgesetzte Verbindung ein aminogeschützes Derivat ist.

4. verfahren nach Anspruch 1, in dem die vorgenannte umgesetzte Verbindung ein Derivat mit geschützten aromatischen Hydroxyl- und Aminogruppen ist.

5. Verfahren nach Anspruch 1, in dem die vorgenannte umgesetzte Verbindung eine Verbindung der folgenden allgemeinen Formel ist:

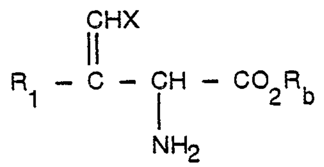 oder 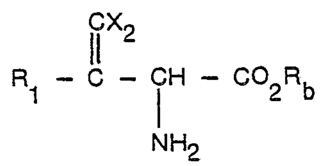

wobei:

$R_b$ einen $C_1$—$C_4$-Alkylrest bedeutet;

X ein Fluor-, Chlor- oder Bromatom ist und

$R_1$ die folgende Bedeutung hat,

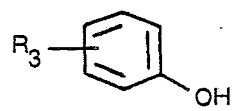

wobei $R_3$ ein Wasserstoffatom oder ein $C_1$—$C_4$-Alkylrest ist; oder ein Derivat davon mit geschützen aromatischen Hydroxyl- und/oder Aminogruppen.

6. Verfahren nach Anspruch 5, in dem die vorgenannte umgesetzte Verbindung ein $C_1$—$C_4$-Alkylester der 2-Amino-4-fluor-3-(3'-hydroxyphenyl)-3-butensäure ist.

7. Verfahren nach Anspruch 5, in dem die vorgenannte umgesetzte Verbindung ein Derivat mit geschützten aromatischen Hydroxylgruppen eines $C_1$—$C_4$-Alkylesters der 2-Amino-4-fluor-3-(3'-hydroxyphenyl)-3-butensäure ist.

8. Verfahren nach Anspruch 5, in dem die vorgenannte umgesetzte Verbindung ein aminogeschütztes Derivat eines $C_1$—$C_4$-Alkylesters der 2-Amino-4-fluor-3-(3'-hydroxyphenyl)-3-butensäure ist.

9. Verfahren nach Anspruch 5, in dem die vorgenannte umgesetzte Verbindung ein Derivat mit geschützen aromatischen Hydroxyl- und mit geschützten Aminogruppen eines $C_1$—$C_4$-Alkylesters der 2-Amino-4-fluor-3-(3'-hydroxyphenyl)-3-butensäure ist.

10. Verfahren nach einem der Ansprüche 1 bis 9, wobei die Hydrolysereaktion unter Verwendung einer starken wäßrigen Säure bei Rückflußtemperatur durchgeführt wird.

11. Verfahren nach Anspruch 10, wobei die Hydrolysereaktion unter Verwendung einer 47 prozentigen wäßrigen Bromwasserstoffsäure bei Rückflußtemperatur durchgeführt wird.

12. Verfahren nach Anspruch 1 zur Herstellung von 2-Amino-3-(3'-hydroxyphenyl)-3-butensäure, 2-Amino-3-(3',4'-dihydroxyphenyl)-3-butensäure oder eines nicht-toxischen pharmakologisch verträglichen Salzes davon zur Verwendung in der Behandlung von Depressionen beim Menschen.

**Revendications pour les Etats contractants: BE CH DE FR IT LI LU NL SE**

1. Composé de formule:

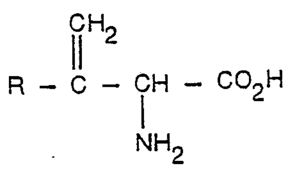 , 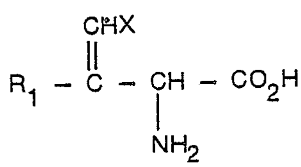 , ou 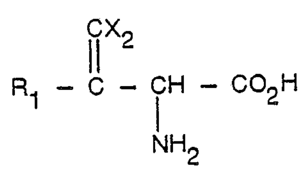

I II III

dans laquelle:

X représente le fluor, le chlore ou le brome;

R représente le group $R_2$ tel que défini ci-après;
$R_1$ est un groupe de formule:

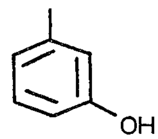 or 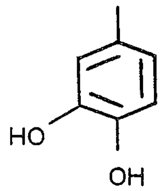

ou représente le groupe $R_2$ tel que défini ci-après;
$R_2$ représente:

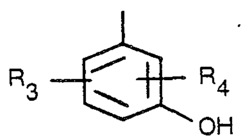 , 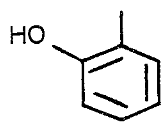 , 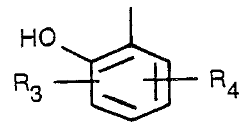 ,

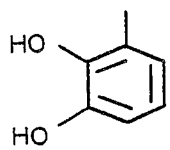 , 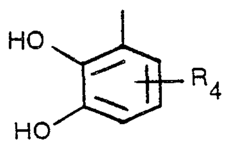 , 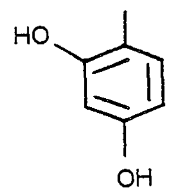 ,

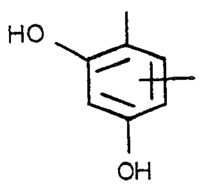 , 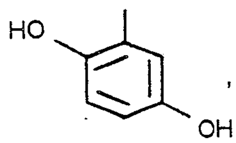 , 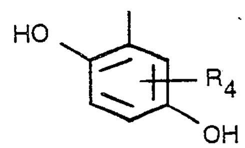 ,

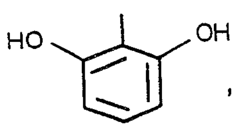 , 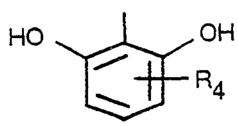 , 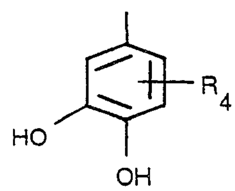 ,

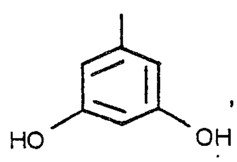 , ou 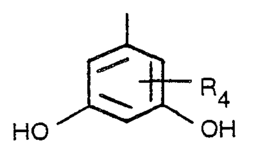 ;

oú $R_3$ représente un atome d'hydrogène, un groupe alkyle en $C_1$—$C_4$ our alcoxy en $C_1$—$C_4$, et $R_4$ représente un groupe alkyle en $C_1$—$C_4$ ou alcoxy en $C_1$—$C_4$,
ou un sel pharmaceutiquement acceptable et non toxique de ce composé.

2. Composé de formule I, tel que revendiqué à la revendication 1.
3. Composé de formule II, tel que revendiqué à la revendication 1.
4. Composé de formule III, tel que revendiqué à la revendication 1.
5. Composé selon l'une des revendications 3 et 4, dans lequel $R_1$ représente:

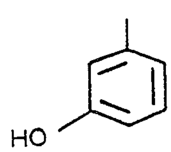 .

6. Composé tel que revendiqué dans l'une des revendications 3 et 4, lequel $R_1$ représente:

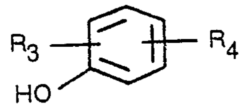 ,

où $R_3$ représente un atome d'hydrogène, un groupe alkyle en $C_1$—$C_4$ ou alcoxy en $C_1$—$C_4$, et $R_4$ représente un groupe alkyle en $C_1$—$C_4$ ou alcoxy en $C_1$—$C_4$.

7. Composé selon la revendication 6, dans lequel $R_3$ représente l'hydrogène.

8. Composé selon l'une quelconque des revendications 3 à 7, dans lequel X représente du fluor.

9. Acide 2-amino-4-fluoro-3-(3'-hydroxyphényl)-3-buténoïque ou un sel pharmaceutiquement acceptable et non toxique de cet acide.

10. Composé selon l'une quelconque des revendications précédentes, destiné à servir à l'inhibition de la monoamine oxydase dans le corps humain.

11. Composé selon l'une quelconque des revendications précédentes, destiné à servir au traitement de la dépression dans le corps humain.

12. Composition pharmaceutique, comprenant un composé tel que revendiqué dans l'une quelconque des revendications précédentes, en mélange ou associé d'une autre façon avec un support, excipient ou diluant pharmaceutiquement acceptable de ce composé.

13. Composition pharmaceutique selon la revendication 12 sous forme de doses unitaires contenant 1 mg à 100 mg dudit composé par dose unitaire.

14. Composition pharmaceutique comprenant (a) un composé selon l'une quelconque des revendications 1 à 11, et (b) un inhibiteur de la décarboxylase de L-aminoacide aromatique.

15. Composition pharmaceutique selon la revendication 14, dans laquelle les substances actives sont en mélange, ou associées autrement, avec un excipient, support ou diluant pharmaceutiquement acceptable.

16. Composé choisi parmi l'acide 2-amino-3-(3'-hydroxyphényl)-3-buténoïque, l'acide 2-amino-3-(3',4'-dihydroxyphényl)-3-buténoïque, ou un sel pharmaceutiquement acceptable et non toxique de ces acides, destiné à servir au traitement d'une dépression dans le corps humain.

17. Composition pharmaceutique comprenant l'acide 2-amino-3-(3'-hydroxyphényl)-3-buténoïque ou un sel pharmaceutiquement acceptable et non toxique de cet acide, en mélange, ou associé autrement, avec un support, excipient ou diluant pharmaceutiquement acceptable.

18. Composition pharmaceutique comprenant l'acide 2-amino-3-(3',4'-dihydroxyphényl)-3-buténoïque ou un sel pharmaceutiquement acceptable et non toxique de cet acide, en mélange, ou en association autrement, avec un excipient, support ou diluant pharmaceutiquement acceptable.

19. Composition pharmaceutique, comprenant (a) l'acide 2-amino-3-(3'-hydroxyphényl)-3-buténoïque, ou un sel pharmaceutiquement acceptable et non toxique tde cet acide, et (b) un inhibiteur de la décarboxylase de L-aminoacide aromatique.

20. Composition pharmaceutique, comprenant (a) l'acide 2-amino-3-(3',4'-dihydroxyphényl)-3-buténoïque ou un sel pharmaceutiquement acceptable et non toxique de cet acide, et (b) un inhibiteur de la décarboxylase de L-aminoacide aromatique.

21. Composition pharmaceutique selon l'une des revendications 19 et 20, dans laquelle les substances actives sont en mélange, ou associées autrement, avec un support, excipient ou diluant pharmaceutiquement acceptable.

22. Composition pharmaceutique selon l'une quelconque des revendications 14, 15, 19, 20 et 21, dans laquelle l'inhibiteur de décarboxylase de L-aminoacide aromatique est la carbidopa, le benzérazide, ou un acide 2-amino-2-(monofluorométhyle ou difluorométhyl)-3-(monohydroxyphényle ou dihydroxyphényl)propionique.

23. Procédé pour préparer un composé selon la revendication 1, qui comprend l'hydrolyse d'un composé de formule:

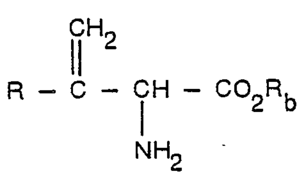 , 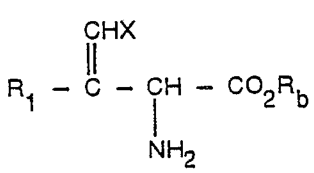 , ou 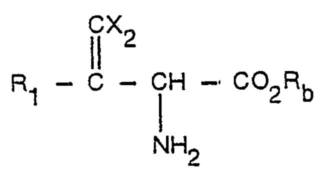

dans lesquelles:

R, $R_1$ et X sont tels que définis à la revendication 1, et

$R_b$ représente un groupe alkyle en $C_1$—$C_4$;

ou un dérivé de ce composé, à groupe hydroxyle aromatique protégé et/ou à groupe amino protégé; et, si nécessaire, l'enlèvement de façon connu du ou des groupe(s) protecteur(s), en même temps que la réaction d'hydrolyse ou après celle-ci.

**Revendications pour l'Etat contractant: AT**

1. Procédé pour préparer un composé de formule:

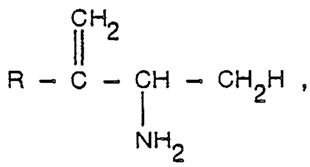 , 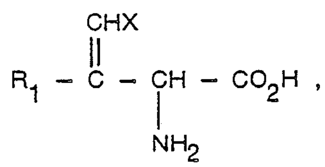 , ou 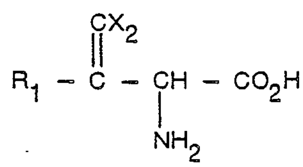

I II III

dans laquelle:

X représente le fluor, le chlore ou le brome;

R représente le groupe $R_2$, tel que défini ci-après;

$R_1$ représente un groupe de formule:

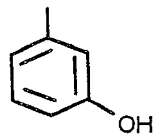 ou 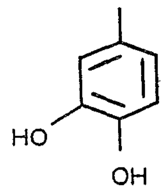

ou le groupe $R_2$ selon la définition ci-après;

$R_2$ représente:

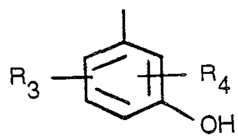 , 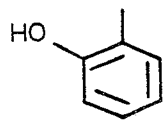 , 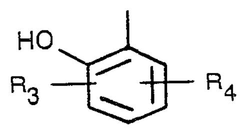 ,

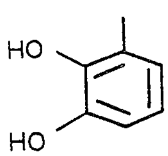 , 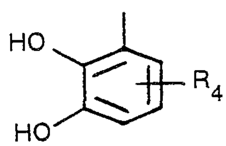 , 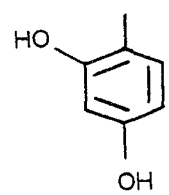 ,

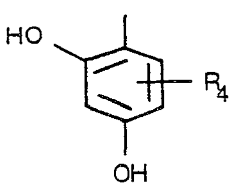 , 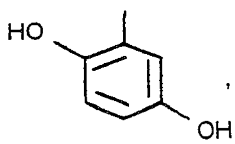 , 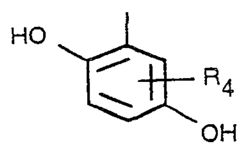 ,

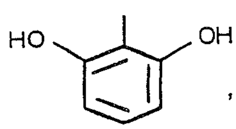 , 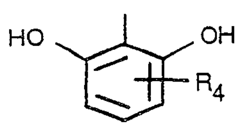 , 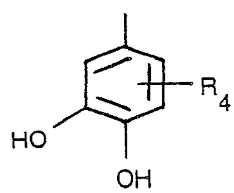 ,

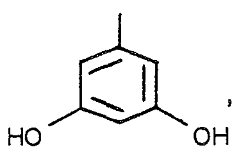 , ou 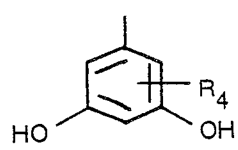 ;

$R_3$ représente un atome d'hydrogène, un groupe alkyle en $C_1$—$C_4$ ou alcoxy en $C_1$—$C_4$, et

$R_4$ représente un groupe alkyle en $C_1$—$C_4$ ou alcoxy en $C_1$—$C_4$ ou un sel pharmaceutiquement acceptable et non toxique de ce composé, qui comprend l'hydrolyse d'un composé de formule:

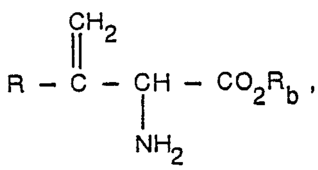 , 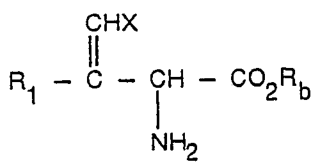 , ou 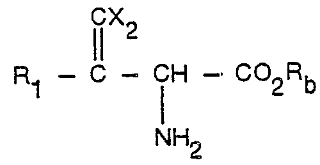

dans lesquelles:

R, $R_1$ et X sont tels que définis di-dessus et

$R_b$ représente un groupe alkyle en $C_1$—$C_4$;

ou d'un dérivé à groupe hydroxyle aromatique protégé et/ou à groupe amino protége; et, si nécessaire, l'enlèvement d'une façon connue du ou des groupe(s) protecteur(s), en même temps que la réaction d'hydrolyse ou après celle-ci.

2. Procédé selon la revendication 1, dans lequel ledit composé traité est un dérivé à groupe hydroxyle aromatique protége.

3. Procédé selon la revendication 1, dans lequel le composé traité est un dérivé à groupe amino protété.

4. Procédé selon la revendiction 1, dans lequel ledit composé traité est un dérivé à groupe hydroxyle aromatique protégé et à groupe amino protégé.

5. Procédé selon la revendication 1, dans lequel ledit composé traité est un composé la formule:

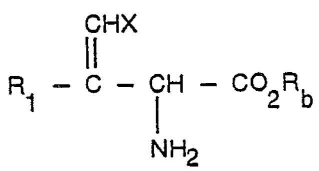 ou 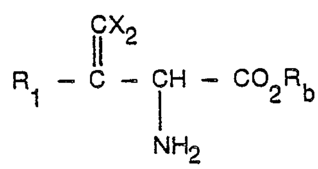

dans lesquelles:

$R_b$ représente un groupe alkyle en $C_1$—$C_4$,

X représente le fluor, le chlore ou le brome, et

$R_1$ représente:

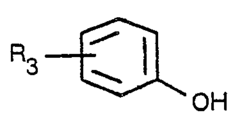 .

où $R_3$ représente un atome d'hydrogène ou un groupe alkyle en $C_1$—$C_4$; ou un dérivé à groupe hydroxyle aromatique protégé et/ou à groupe amino protégé.

6. Procédé selon la revendication 5, dans lequel ledit composé traité est un ester d'alkyle en $C_1$—$C_4$ de l'acide 2-amino-4-fluoro-3-(3'-hydroxyphényl)-3-buténoïque.

7. Procédé selon la revendication 5, dans lequel ledit composé traité est un dérivé à groupe hydroxy aromatique protégé, d'un este d'alkyle en $C_1$—$C_4$ de l'acide 2-amino-4-fluoro-3-(3'-hydroxyphényl)-3-buténoïque.

8. Procédé selon la revendication 5, dans lequel ledit composé traité est un dérivé, à groupe amino protégé, d'un ester d'alkyle en $C_1$—$C_4$ de l'acide 2-amino-4-fluoro-3-(3'-hydroxyphényl)--3-buténoïque.

9. Procédé selon la revendication 5, dans lequel ledit composé traité est un dérivé à groupe hydroxyle aromatique protégé et à groupee amino protégé d'un ester d'alkyle en $C_1$—$C_4$ de l'acide 2-amino-4-fluoro-3-(3'-hydroxyphényl)-3-buténoïque.

10. Procédé selon l'une quelconque des revendications 1 à 9, dans lequel on effectue la réaction d'hydrolyse en utilisant un acide fort aqueux, à la température du reflux.

11. Procédé selon la revendication 10, dans lequel on effectue la réaction d'hydrolyse en utilisant du bromure d'hydrogène aqueux à 47%, à la température du reflux.

12. Procédé selon la revendication 1 pour préparer l'acide 2-amino-3-(3'-hydroxyphényl)-3-buténoïque, l'acide 2-amino-3-(3',4'-dihydroxyphényl)-3-buténoïque, ou un sel pharmaceutiquement acceptable et non toxique de ces acides, destinés à servir au traitement d'une dépression dans le corps humain.